# EUROPEAN PATENT SPECIFICATION

(11) **EP 3 373 928 B1**
(45) Date of publication and mention of the grant of the patent: **06.10.2021**
(21) Application number: 16864840.0
(22) Date of filing: 08.11.2016
(51) Int. Cl.: A61K 8/34, A61K 31/32, A61Q 19/00, A61K 8/81, A61K 8/60, A61K 8/38, A61Q 19/10, A61K 9/00, A61K 31/327, A61P 17/00, A61P 17/10

(54) **CLEANSER COMPOSITIONS COMPRISING BENZOYL PEROXIDE**
BENZOYLPEROXID ENTHALTENDE REINIGUNGSMITTEL
COMPOSITION NETTOYANTE COMPRENANT DU PEROXYDE DE BENZOYLE

(30) Priority: 09.11.2015 US 201562252724 P
(43) Date of publication of application: 19.09.2018
(73) Proprietor: GlaxoSmithKline Consumer Healthcare Holdings (US) LLC, Wilmington, DE 19808 (US)
(72) Inventor: SHALVIRI, Alireza, Warren, NJ 07059 (US); CLARKE, Martyn, J., Zebulon, NC 27597 (US)
(74) Representative: Goff, Dawn Caroline
(86) International application number: PCT/US2016/060924
(87) International publication number: WO 2017/083266

(56) References cited:
- WO-A1-2014/076135
- US-A- 6 117 843
- US-A1- 2008 131 381
- US-A1- 2009 226 390
- US-A1- 2014 154 342
- US-A1- 2014 271 670
- US-A1- 2015 306 004
- US-A1- 2017 128 407
- US-B1- 6 627 612
- US-B1- 9 144 434
- US-B2- 8 563 535
- US-B2- 9 107 844

## Description

### FIELD OF THE INVENTION

The present invention relates to novel Benzoyl Peroxide cleansers and washes which are gentle to the skin, stable and do not contain potentially irritating surfactants.

### BACKGROUND OF THE INVENTION

Acne vulgaris (or simply acne) is a common skin condition affecting an estimated 650 million people, or 9.4% of the population, worldwide (Vos et al., Lancet, 380(9859):2163-2196, 2012). The condition, characterized by areas of skin with seborrhea, comedones, papules, nodules, pimples, and possible scarring, often occurs in adolescences, but may persists much further into adulthood (James. N Engl J Med, 352(14):1463-1472, 2005).

Adolescence is a period of high social insecurity, and the appearance of and potential scarring from acne often result in psychological issues such as reduced self-esteem, depression, or, in extreme cases, suicide (Goodman. Aust Fam Physician, 35(7):503-504, 2006; Purvis et al. J Paediatr Child Health, 42(12):793-796, 2006).

Acne is a multi-factorial disease with increased sebum production, follicular hypercornification, colonization with *Propionibacterium acne (P.acne),* lymphocytic inflammatory response, and the *P.acne* biofilm being identified as the main causes (E. A. Tanghetti et al., Dermatologic Clinics. 27:17-24 (2009); and J.E.Fulton, et al., Journal of Cutaneous Pathology. 1:191-200 (1974)).

Acne treatments work by reducing sebum production by sebocytes, speeding up cell turnover, fighting bacterial infection, reducing inflammation, or some combination of these strategies. Treatment of acne tends to be long and primarily focuses on the use of retinoids, benzoyl peroxide (BPO), and antibacterials - particularly oral tetracyclines and topical clindamycin.

Benzoyl peroxide (BPO) has proven to be effective in the treatment of mild to moderate acne (E.A. Tanghetti, supra; M. Sagransky et al., and J.Q. Del Rosso, Cutis, 82: 336-342 (2008)). Benzoyl peroxide products come in many different dosage forms and in combination prescription products. Depending upon the % w/w amount of BPO present, the product may be a prescription product or be available to the consumer directly.

In the consumer space, there are conventional "leave-on" and harsh BPO containing cleansers, such as PanOyxl® 10 Acne Foaming wash and PanOxyl® 4 Acne Creamy wash, produced by GSK Consumer HealthCare.

Harsh surfactants and excessive cleansing may even exacerbate acne. It has been shown that mild moisturizing cleansers may be more effective in reducing negative characteristics such as itching, dryness, and oiliness (G.Craig et al., SKINmed:Dermatology for the Clinician 4:370-396 (2005). Hence, there is a need for an ultra-mild moisturizing BPO skin cleanser in patients with acne prone sensitive skin. Suitably, a cleaner that has a gentle moisturizing effect, which foams and leaves a clean after feel is desired by consumers.

Document WO2014076135 discloses compositions based on BPO. The composition of ex. 13 comprises BPO, water, humectant and a thickening agent (magnesium aluminium silicate). D1 does not disclose compositions comprising alkylglycoside sulfonate, an alkylpolyglycoside sulfonate, or an alkylpolyglycoside phosphate.

Thus, while there are products available for the consumer, there is still a need for additional products, including those in lower dosage strengths, those which have improved shelf life, and/or potentially reduced irritation as an inventive addition to acne treatment regimens.

### SUMMARY OF THE INVENTION

The present invention relates to a topical pharmaceutical or cosmetic cleanser composition comprising
a) benzoyl peroxide present in an amount from about 2 % to about 20% w/w;
b) water;
c) at least one anionic surfactant which is an alkylglucoside (AG) derivative surfactant selected from an alkylglycoside sulfonate, an alkylpolyglycoside sulfonate, or an alkylpolyglycoside phosphate;
d) at least one humectant;
e) a thickening agent; and
f) optionally at least one dermatologically acceptable excipient.

In one embodiment the thickening agent is a carbomer. In an embodiment, the carbomer is Carbopol 980 NF. In another embodiment the thickening agent is an acrylate copolymer. In an embodiment the acrylate copolymer is polyacrylate crosspolymer-6. In another embodiment the thickening agent is a combination of an acrylate copolymer and a carbomer. In an embodiment the acrylate copolymer is polyacrylate crosspolymer-6 and the carbomer is the carbomer is Carbopol 980 NF.

In one embodiment the humectant is glycerol or sorbitol, or a combination or mixture thereof. In one embodiment the humectant is glycerol. In another embodiment, the humectant is sorbitol. In another embodiment, the humectant is a mixture of glycerol and sorbitol.

In one embodiment, the humectant is glycerol or sorbitol, or a combination or mixture thereof and the thickening agent is an acrylate copolymer, such as polyacrylate crosspolymer-6.

In one embodiment the at least one anionic surfactant is an alkylglucoside (also referred to herein as 'AG') derivative selected from Sodium Laurylglucoside Hydroxypropylsufonate, Sodium Decylglucoside Hydroxypropylsufonate, Sodium Hydroxypropylphosphate Laurylglucoside Crosspolymer, Sodium Hydroxypropylphosphate Decylglucoside Crosspolymer, or a combination or mixture thereof. In an embodiment, the AG derivative surfactant is Sodium Laurylglucoside Hydroxypropylsufonate or Sodium Decylglucoside Hydroxypropylsufonate, or a mixture thereof. In another embodiment the AG derivative surfactant is Sodium Hydroxypropylphosphate Laurylglucoside Crosspolymer, or Sodium Hydroxypropylphosphate Decylglucoside Crosspolymer, or a mixture thereof. In another embodiment the AG derivative surfactant is Sodium Laurylglucoside Hydroxypropylsufonate and Sodium Hydroxypropylphosphate Laurylglucoside Crosspolymer.

In another embodiment of the invention, the composition further comprises an additional surfactant selected from another anionic surfactant, or a non-ionic surfactant. In one embodiment the anionic surfactant is selected from sodium C14-16 Olefin sulfonate, disodium PEG-12 Dimethicone Sulfosuccinate, ammonium lauryl sulfate, sodium lauroyl methyl isoethionate , Sodium C14-C17 sec-alkyl sulfonate, or disodium laureth sulfosuccinate, and mixtures thereof. In another embodiment the non-ionic surfactant is selected from decyl glucoside or coco glycoside or a mixture thereof.

In one embodiment the composition comprises at least one AG derivative surfactant selected from an alkylglycoside sulfonate, an alkylpolyglycoside sulfonate or an alkylpolyglycoside phosphate, at least on anionic surfactant and at least one non-ionic surfactant.

In one embodiment the additional dermatologically acceptable excipient is selected from a pH adjusting agent, co-emulsifier, a chelating agent, a preservative, a co-solvent, a penetration enhancer, a fragrance, a colorant, and mixtures thereof.

In an embodiment the co-emulsifier is a fatty acid. In an embodiment, the fatty acid is behenic acid or stearic acid, or a combination or mixture thereof.

In an embodiment the pH adjusting agent is sodium or potassium hydroxide.

In one embodiment of the disclosure, there is provided a topical pharmaceutical or cosmetic cleanser composition comprising
a) benzoyl peroxide present in an amount from about 2.0 to about 20% w/w;
b) water;
c) at least one anionic surfactant which is an alkylglucoside (AG) derivative surfactant selected from an alkylglycoside sulfonate, an alkylpolyglycoside sulfonate, or an alkylpolyglycoside phosphate;
d) at least one humectant;
e) a thickening agent; and
f) optionally at least one dermatologically acceptable excipient for use in treating acne.

In one embodiment of the disclosure, there is provided a topical pharmaceutical or cosmetic cleanser composition comprising
a) benzoyl peroxide present in an amount from about 2.0 to about 20% w/w;
b) water;
c) at least one anionic surfactant which is an alkylglucoside (AG) derivative surfactant selected from an alkylglycoside sulfonate, an alkylpolyglycoside sulfonate, or an alkylpolyglycoside phosphate;
d) at least one humectant;
e) a thickening agent; and
f) optionally at least one dermatologically acceptable excipient for use in killing *P. acnes* in a subject in need thereof.

In one embodiment of the disclosure, there is provided a topical pharmaceutical or cosmetic cleanser composition comprising
a) benzoyl peroxide present in an amount from about 2.0 to about 20% w/w;
b) water;
c) at least one anionic surfactant which is an alkylglucoside (AG) derivative surfactant selected from an alkylglycoside sulfonate, an alkylpolyglycoside sulfonate, or an alkylpolyglycoside phosphate;
d) at least one humectant;
e) a thickening agent; and
f) optionally at least one dermatologically acceptable excipient for use in treating a follicular disease in a subject in need thereof.

### BRIEF DESCRIPTION OF THE DRAWINGS

Figure 1 shows the irritancy potential of selected surfactants in a HaCaT Cell viability assay.
Figure 2 shows in vitro skin irritancy of certain 2.5% BPO formulations in an HaCaT cell viability assay.
Figure 3 shows in vitro skin irritancy of certain 10% BPO formulations in an HaCaT cell viability assay.
Figure 4 shows the pH values of the 2.5% and 10% formulations on storage.
Figure 5 shows stability data of 2.5% BPO wash formulations with sugar based surfactants (formulation #4) as compared to non-sugar based surfactant containing formulations (formulations #22 and #23).
Figure 6 shows stability of Curoxyl 42 (BPO) in binary mixtures of various surfactants using cumulative benzoic acid levels (% BPO) as the stability marker. The concentration of all surfactants was fixed at 6% w/w active. The least amount of BPO degradation, in these binary studies, is demonstrated by the sugar based surfactants.
Figure 7 demonstrates the effect of storage under accelerated conditions (30 C/65% RH at 3 months) on the viscosity of the 2.5% and 10% formulations as shown therein.

### DETAILED DESCRIPTION OF THE INVENTION

There are lots of problems surrounding formulating BPO into a topical pharmaceutical product. Some of them include its chemical stability on storage and its potential for interacting with other ingredients in the formulation.

The stability of BPO in topical pharmaceutical products is partly dependent on the types of excipient and solvent present in the formulation. In solution, BPO can degrade at temperatures below 40°C because of the extreme instability of its peroxide bond, whose free energy content is markedly lower (∼30 kcal/mol) than that of a (C-C) bond (∼83 kcal/mol).

Foaming BPO wash formulations are a popular choice for treatment of mild to moderate acne with the consumers. Traditionally, these formulations contain substantial amount of surfactants in order to provide foaming and cleansing, removing oils and dirt from the skin pores, which are perceived as one of the causes of acne by majority of consumers. However, a majority of the surfactants used in consumer products are known to increase the degradation rate of BPO and cause skin irritation.

Accelerated surfactant induced BPO degradation is due to increased BPO solubility, and formation of surfactant's free radicals which can accelerate BPO degradation. As a result, formulating low BPO contents wash formulations that would comply with EU, Brazilian and other countries guidelines are very challenging. While low dose, e.g. 2.5% BPO products are available in the US due to increased flexibility on BPO degradation, they are not available in the rest of the world.

The efficacy of benzoyl peroxide is associated with its decomposition when it is placed in contact with the skin. Thus, it is the oxidizing properties of the free radicals produced during this decomposition which lead to the desired effect. In order to maintain optimal efficacy for benzoyl peroxide, it is important to prevent its decomposition before use, i.e. during storage. However, benzoyl peroxide is an unstable chemical compound, this instability making it difficult to be formulated into finished products having any reasonable shelf life.

The solubility and stability of benzoyl peroxide was studied by Chellquist et al. in ethanol, propylene glycol and various mixtures of polyethylene glycol 400 (PEG 400) and water (Chellquist E. M. and Gorman W. G., Pha/. m. Res.,1992, vol. 9: 1341-1346). Chellquist et al. states that the stability of benzoyl peroxide is greatly influenced by the chemical composition of the formulation and by the storage temperature. As noted, benzoyl peroxide is extremely reactive and degrades in solution at low temperature on account of the instability of its peroxide bond. The authors found that benzoyl peroxide in solution degrades more or less quickly in all the solvents studied as a function of the type of solvent and of its concentration. Degradation, such as described therein, does not enable the preparation of a product intended for sale which needs sufficient shelf life for product, shipping, storage and use by the consumer. It is known that benzoyl peroxide is more stable in water and propylene glycol when it is in suspension (i.e. in dispersed form), since it is not degraded after 90 days of storage in these solvents.

Even at below 40°C temperatures, BPO is inherently unstable in solution due to low energy of its O-O bond. The rate of thermal degradation increases exponentially with an increase in temperature. In pharmaceutical formulations, the rate of BPO degradation depends on storage temperature, solvent type, formulation components, BPO solubility, and the pH (See K. Nozaki et al., Int'l J Am Chem Soc., 68:1686-1692 (1946).

A BPO degradation pathway is shown in Scheme 1 below. Initially, BPO degrades thermally to form benzoate-derived free radicals. These radicals can then propagate the reaction by reacting either with another BPO molecule, with the solvent, or with other formulation components in the solution (e.g. surfactants) to form solvent radicals and other benzoate radicals which in turn react with BPO. The reaction rate is dependent on the reactivity of generated free radicals, and the number of propagation reactions per initiation reaction determines the chain length. The reaction is terminated when two radicals couple together.

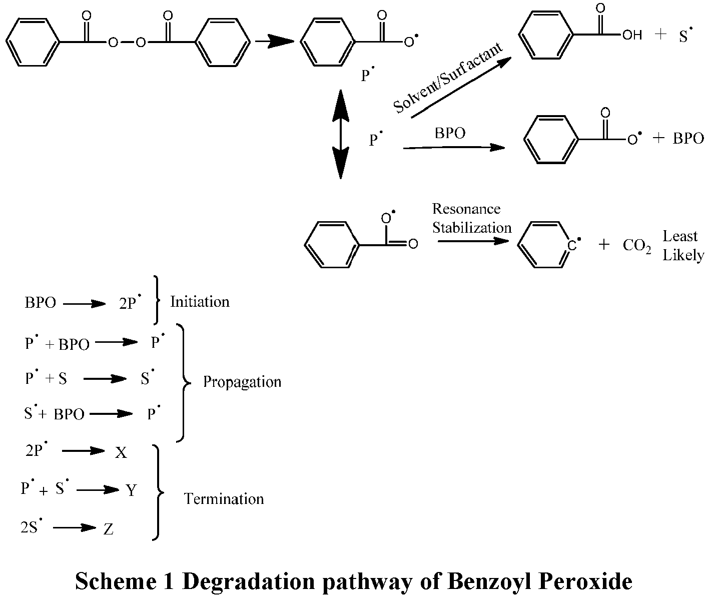

Therefore to limit the problem of rapid instability of benzoyl peroxide in solution, it has proven advantageous to use benzoyl peroxide in its dispersed form. However, this type of formulation is not entirely satisfactory as degradation of benzoyl peroxide in the finished product is still observed.

In addition to the above challenges to develop a BPO containing product, when that product is a cleanser formulation, product mildness also becomes an issue. The current wash products on the market generally contain soap bases with high amounts of potentially irritating surfactants, such as Potassium Lauryl Sulfate (PLS) and/or Sodium Lauryl Sulfate (SLS). These surfactants are not really suitable for patients with sensitive skin.

To make a cleanser or wash formulation (as used interchangeably herein), two of the necessary components therefore will include benzoyl peroxide and a surfactant. The composition will also comprise water and other dermatologically acceptable excipients as well as will be further described herein.

Suitably the BPO can be coarse or milled. In one embodiment it is milled. The particle size (the D90 cut) is suitably <60 µm. In an embodiment the D90 cut is suitably less than <30 µm. In another embodiment the particle size D90 cut is less than <20µm. Milling however, results in increased manufacturing times. Both coarse or milled BPO can be commercially purchased.

Benzoyl peroxide may be used either in free form or in an encapsulated form, for example in a form adsorbed onto or absorbed into any porous support. It may be, for example, benzoyl peroxide encapsulated in a polymeric system consisting porous microspheres, for instance microsponges sold under the name Microsponges P009A Benzoyl peroxide the company Cardinal Health.

Suitably, one commercially available micronized benzoyl peroxide product for use herein is an aqueous gel dispersion, such as Curoxyl® 42, having an INCI name of Benzoyl peroxide Gel, USP. Curoxyl® 42 is a is an aqueous based, micronized benzoyl peroxide dispersion (40%) in the form of a gel, commercially available from Vantage, NJ, USA. Other commercially available BPO containing excipients include Luperox A75 (Arkema®), a coarsely ground mixture of 75% BPO and 25% water, Luperox SC, (Arkema®), a premicronized BPO slurry, and Salsphere (Salvona®) an encapsulated BPO.

Curoxyl®42 contains micronized BPO particles with the average particle size of 5-7 µm. A smaller particle size is believed to better penetrate into pores and attack *P. Acnes* bacteria. It may also provide for a smoother application, and greater efficacy in comparison to traditional BPO sources (15-30 µm). Use of a micronized BPO does not require in-process milling, and thus offers significant reduction in production time and costs.

The amount of BPO useful in a cleanser or wash of the present invention is from about 1 to about 20% w/w of the composition. In another embodiment, the BPO is present in an amount of about 2 to 20%. In one embodiment, the amount of BPO present is from about 2.5 to about 10% w/w. In one embodiment, the amount of BPO present is from about 4.0 to about 10% w/w. In another embodiment, the amount of BPO present is about 2.0%, 2.5%, 3 %, 4%, 5%, 6%, 7%, 8%, 9% and 10% w/w. In one embodiment, the amount of BPO present is 2.5%, 3.0%, 4%, 8% and 10% w/w. In one embodiment, the amount of BPO present is 4%, 8% or 10%. In one embodiment, the amount of BPO present is 2.5%. In one embodiment, the amount of BPO present is 4%. In one embodiment, the amount of BPO present is 8%. In one embodiment, the amount of BPO present is 10%.

In an embodiment the product is a very low strength BPO containing wash product (i.e., 2.0-3.0%). In another embodiment, the product is a low strength BPO containing wash product such as 4.0%. In another embodiment, the product is a higher strength BPO containing wash product such as 10.0 %. In one aspect the product is a low strength BPO product that has a long shelf life, such as 24 months or greater. In one aspect the product is a higher strength BPO product that has a long shelf life, such as 18months, or 24 months or greater. In one aspect the product is a very low strength BPO product that has a long shelf life, such as 18 months or greater.

### Surfactants

Surfactants are one of the most vital components of BPO cleanser compositions. They provide foaming, cleansing, and removal of dirt and excess oil from the skin. An ideal surfactant system should be esthetically acceptable, non-toxic, non-irritating, environmentally friendly and provide sufficient foaming and cleansing while being compatible with BPO. It is a common practice to combine surfactants of different classes into a cleansing system to achieve optimum foaming, cleansing and mildness.

A majority of commercially available washes contain some combination of anionic, amphoteric and non-ionic surfactants. Cationic surfactants are less commonly used in the wash products as these surfactants foam less and are generally more irritating to the skin.

Use of surfactants in a BPO wash formulation can be challenging due to increased BPO degradation, as well as increased skin irritation. It has surprisingly been found that particularly in the low dose BPO containing wash formulations that the degradation of BPO can be significantly reduced by use of the AG derivative surfactants. This reduction of BPO degradation allows a low dose formulation to be shelf stable for long enough periods of time to be able to meet EP and other countries degradation limits upon storage, etc.

Surfactants may be classified, according to their structure, under the generic terms "ionic" (anionic, cationic or amphoteric) or "non-ionic". Non-ionic surfactants are surfactants that do not dissociate into ions in water and are thus insensitive to pH variations. As used herein, a surfactant is a compound that lowers the surface tension between two liquids or between a liquid and a solid. Surfactants may also act as detergents, wetting agents, emulsifiers, foaming agents, and dispersants. As further used herein an emulsifier is equivalent to a surfactant.

The topical pharmaceutical compositions of the present invention comprise at least one AG derivative surfactant. That AG derivative surfactant is selected from an alkylglycoside sulfonate, an alkylpolyglycoside sulfonate or an alkylpolyglycoside phosphate. In an embodiment, the surfactant component is a mixture of two or more surfactants, including the at least one AG derivative surfactant.

Suitably, the surfactant component is present in the composition in an amount from about 1% to about 25% by weight, based on the total weight of the composition. In another embodiment, the surfactant component is present in the composition in an amount from about 5% to about 15% by weight, based on the total weight of the composition.

The AG derivative surfactant is suitably present in an amount of about 1 to about 20% w/w, based on the total weight of the composition. In another embodiment, the AG derivative surfactant is present in an amount of about 1 to about 10% w/w. In another embodiment the AG derivative is present in an amount of about 1 to about 5% w/w, based upon the total weight of the composition.

Suitably when there is a second surfactant in the composition, and it is a non-ionic surfactant, it is present in an amount from about 1 to about 10% w/w. In another embodiment it is present in an amount from about 1 to about 5% w/w, based on the total weight of the composition. Suitably when the second surfactant in the composition, and it is an amphoteric surfactant, it is present in an amount from about 1 to about 5% w/w. In another embodiment, is present in an amount from about 1 to about 2% w/w, based on the total weight of the composition.

When the composition contains as the surfactant component the AG derivative, a non-ionic surfactant and an amphoteric surfactant the three surfactants are optimally present in an amount of about 1 to about 15%: 1 to about 5%: and 1 to about 5% w/w respectively, based on the total weight of the composition.

In one embodiment, the surfactant component comprises a second anionic surfactant. Non limiting examples of the second anionic surfactant include but are not limited to sodium lauryl sulfate (Texapon K12 P PH® by Cognis), ammonium or triethanolamine lauryl sulfate, sodium lauryl ether sulfate (such as the sodium laureth sulfate sold under the name Texapon N70®, by Cognis), magnesium, ammonium or TEA (triethylamine) lauryl ether sulfate, sodium lauroyl sarcosinate (sold under the name Protelan LS9011® by the company Zschimmer & Schwartz), sodium olefin sulfonates, sodium sulfoacetates, sulfosuccinates or taurates, sodium cocoyl glutamate & disodium cocoyl glutamate (sold under the name Amisoft CS-22® by Ajinomoto).

The anionic surfactants for use herein are the alkylpolyglucoside (AG) derivative as defined in claim 1. More specifically the AG derivative is an alkylpolyglycoside sulfonate or a poly alkylpolyglycoside phosphate surfactant. These include, but are not limited, to Sodium Laurylglucoside Hydroxypropylsufonate (Suganate 160NC, Colonial Chemicals, TN, USA); Sodium Decylglucoside Hydroxypropylsufonate (SugaNate 100NC Colonial Chemicals, TN, USA); Sodium Hydroxypropylphosphate Laurylglucoside Crosspolymer (PolySugaPhos 1200p, Colonial Chemicals, TN, USA), and Sodium Hydroxypropylphosphate Decylglucoside Crosspolymer (PolySugaPhos 1000P, Colonial Chemicals, TN, USA).

In one embodiment, the AG derivative surfactant is at least one alkylglycoside sulfonate or an alkylpolyglycoside sulfonate surfactant. That surfactant is suitably selected from a hydroxypropylsufonate derivative. Suitable hydroxypropylsufonate derivatives include but are not limited to Sodium Laurylglucoside Hydroxypropylsufonate, Sodium Decylglucoside Hydroxypropylsufonate, or a mixture thereof. In one embodiment the at least one hydroxypropylsufonate derivative is Sodium Laurylglucoside Hydroxypropylsufonate. In another embodiment the at least one hydroxypropylsufonate derivative is Sodium Decylglucoside Hydroxypropylsufonate.

In another embodiment the AG derivative surfactant is at least one alkylpolyglycoside phosphate surfactant. That surfactant is suitably selected from a hydroxypropylphosphate derivative. Suitable hydroxypropylphosphate derivatives include but are not limited to Sodium Hydroxypropylphosphate Laurylglucoside Crosspolymer, Sodium Hydroxypropylphosphate Decylglucoside Crosspolymer, or a mixture thereof. In one embodiment the at least one hydroxypropylphosphate derivative is Sodium Hydroxypropylphosphate Laurylglucoside Crosspolymer. In another embodiment the at least one hydroxypropylphosphate derivative is Sodium Hydroxypropylphosphate Decylglucoside Crosspolymer.

In another embodiment, the AG derivative surfactant component comprises at least one hydroxypropylsufonate derivative and at least one hydroxypropylphosphate derivative.

In Table 1 below, representative chemical structures of suitable AG derivative surfactants are provided.

| **Surfactants** | | **Structure** |
|---|---|---|
| **INCI Name** | **Trade Name** | |
| Sodium Laurylglucoside hydroxypropyl sulfonate | Suganate 160NC | 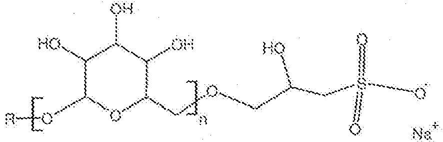 |
| Sodium Decylglucoside hydroxypropyl sulfonate | Suganate 100NC | 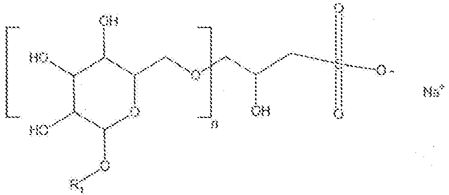 |
| Sodium Hydroxypropylphosphate Laurylglucoside | PolySuga phos 1200p | 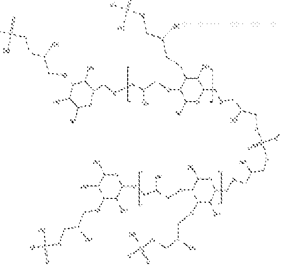 |
| Sodium Hydroxypropylphosphate Decylglucoside | PolySuga phos 100p | 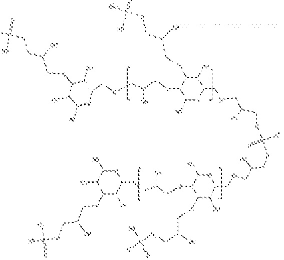 |
| Sodium Bis-Hydroxyethylglycinate Coco-Glucosides Crosspolymer L: Sodium Bis-Hydroxyethylglycinate Lauryl-Glucosides Crosspolymer | Polysuga glycinate C | 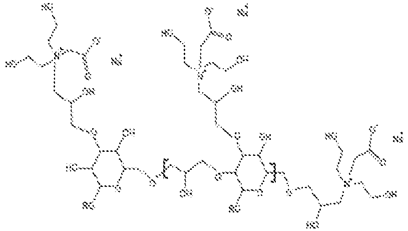 |

In one embodiment, the surfactant component comprises a second anionic surfactant. Potentially, there are many anionic surfactants suitable for use herein. In one embodiment, the second anionic surfactant for use herein includes sodium C14-16 Olefin sulfonate (Colonial AOS), disodium PEG-12 Dimethicone Sulfosuccinate (Colmate SI), ammonium lauryl sulfate (Colonial ALS), sodium lauroyl methyl isoethionate (Iselux LQ-CLR), Sodium C14-C17 sec-alkyl sulfonate (Hostapur SAS60), and disodium laureth sulfosuccinate (Colmate DSLS), and mixtures thereof.

One aspect of the invention is where the surfactant component comprises
a) the at least one anionic surfactant which is an alkylglucoside (AG) derivative surfactant selected from an alkylglycoside sulfonate, an alkylpolyglycoside sulfonate or a alkylpolyglycoside phosphate; and the alkylglycoside sulfonate or alkylpolyglycoside sulfonate is a hydroxypropyl sulfonate derivative and the alkylpolyglycoside phosphate is a hydroxypropylphosphate derivative, or a mixture thereof; and
b) a second anionic surfactant selected from disodium PEG-12 Dimethicone Sulfosuccinate, ammonium lauryl sulfate, sodium lauroyl methyl isoethionate, Sodium C14-C17 sec-alkyl sulfonate, or disodium laureth sulfosuccinate, and combination or mixtures thereof.

Another aspect of the invention is where the surfactant component comprises
a) at least one anionic surfactant which is an alkylpolyglucoside (AG) derivative surfactant selected from an alkylglycoside sulfonate, an alkylpolyglycoside sulfonate or a poly alkylpolyglycoside phosphate; and
b) at least one non-ionic surfactant.

In one embodiment the AG alkylglycoside sulfonate derivative is sodium laurylglucoside hydroxypropyl sulfonate. In one embodiment the non-ionic surfactant is decyl glucoside. In another embodiment the non-ionic is coco glucoside. In another embodiment the non-ionic surfactant is a mixture of decyl glucoside and coco glycoside.

In an embodiment the non-ionic surfactant is decyl glucoside (Plantaren 2000 NUP) or coco glycoside (Pantacare 818UP). In one embodiment, the non-ionic surfactant is decyl glucoside. In another embodiment, the non-ionic surfactant is coco glycoside. In another embodiment the non-ionic surfactant is a mixture of decyl glucoside and coco glucoside.

In one embodiment the AG derivative is Sodium Hydroxypropylphosphate Laurylglucoside Crosspolymer and the non-ionic surfactant is decyl glucoside. In another embodiment the AG derivative is Sodium Hydroxypropylphosphate Laurylglucoside Crosspolymer and the non-ionic is coco glucoside. In another embodiment the AG derivative is Sodium Hydroxypropylphosphate Laurylglucoside Crosspolymer, and the non-ionic surfactant is a mixture of decyl glucoside and coco glycoside.

In another embodiment the AG derivative is Sodium Hydroxypropylphosphate Laurylglucoside Crosspolymer, the nonionic surfactant is decyl glucoside and/or coco glycoside and the surfactant component comprises a second anionic surfactant. In one embodiment, the second anionic surfactant is sodium C14-16 Olefin Sulfonate or sodium C14-17 sec-alkyl sulfonate, or a combination or mixture thereof. In one embodiment the second anionic surfactant is sodium C14-16 Olefin Sulfonate.

Another aspect of the invention is the inclusion of a yet another, third class of surfactants in the surfactant component which are the amphoteric surfactants. Suitable amphoteric surfactants include, but are not limited to, Sodium Hydroxyethylglycinate cocoglucoside crosspolymer and sodium bis-hydroxytethylglycinate lauryl glucoside crosspolymer (Poly SugaGlycinate C), Disodium Cocoamphodiacetate (Colateric CDCX-50), Cocamidopropyl PG-Dimonium Chloride Phosphate (Cola lipid C or Arlasilk PTC LQ), Cocamidopropyl Betaine (ChemBetain C), acyl sarcosines and sarcosinates, combinations and mixtures thereof.

The acyl sarcosines include, but are not limited to, Cocoyl Sarcosine, Lauroyl Sarcosine, Myristoyl Sarcosine, Oleoyl Sarcosine, and Stearoyl Sarcosine. These are modified fatty acids, prepared from fatty acids and sarcosine. These modified fatty acids impart greater solubility, crystallinity and acidity compared to the parent fatty acid, i.e., coconut acid, oleic acid, lauric acid, and myristic acid. The acyl sarcosinates include, but are not limited to, Sodium Cocoyl Sarcosinate, Sodium Lauroyl Sarcosinate, Sodium Myristoyl Sarcosinate, Ammonium Cocoyl Sarcosinate, Ammonium Lauroyl Sarcosinate, and their respective salts. In one embodiment the acyl sarcosinate is Sodium Lauroyl Sarcosinate (Crodasinic LS90).

Other non-ionic surfactants for use herein include, but are not limited to, ethoxylated fatty alcohol ethers, PEG castor oils, PEG esters, propylene glycol esters, glyceryl esters and derivatives, polymeric ethers, sorbitan derivatives, emulsifying waxes, and mixtures thereof.

In an embodiment, the non-ionic surfactant is an ethoxylated fatty alcohol ether. Exemplary ethoxylated fatty alcohol ethers include, but are not limited to, steareth-2, steareth-10, steareth-20, steareth-21, steareth-40, steareth-100, beheneth-10, ceteareth-2, ceteareth-3, ceteareth-5, ceteareth-6, ceteareth-10, ceteareth-12, ceteareth-15, ceteareth-20, ceteareth-21, ceteareth-22, ceteareth-25, ceteareth-30, ceteareth-31, ceteareth-32, ceteareth-33, ceteth-2, ceteth-10, ceteth-20, ceteth-23, choleth-24, isoceteth-20, laureth-2, laureth-3, laureth-4, laureth-5, laureth-9, laureth-10, laureth-12, laureth-15, laureth-20, laureth-21, laureth-22, laureth-23, nonoxynol-9, nonoxynol-15, octoxynol-1, octoxynol-9, oleth-2, oleth-5, oleth-10, oleth-20, C20-40 pareth-24 and trideceth-10, and mixtures thereof.

In an embodiment, the non-ionic surfactant is a PEG castor oil. Exemplary PEG castor oils include, but are not limited to, PEG-7 hydrogenated castor oil, PEG-25 hydrogenated castor oil, PEG-30 castor oil, PEG-33 castor oil, PEG-35 castor oil, PEG-36 castor oil, PEG-40 castor oil, PEG-40 hydrogenated castor oil, PEG-50 castor oil, PEG-54 hydrogenated castor oil, PEG-60 castor oil and PEG-60 hydrogenated castor oil, and mixtures thereof.

In an embodiment, the non-ionic surfactant is a PEG ester. Exemplary PEG esters include, but are not limited to, PEG-4 dilaurate, PEG-150 distearate, PEG-12 glyceryl laurate, PEG-120 glyceryl stearate, PEG-6 isostearate, PEG-4 laurate, PEG-8 laurate, PEG-20 methyl glucose sesquistearate, PEG-5 oleate, PEG-6 oleate, PEG-10 oleate, PEG-25 propylene glycol stearate, PEG-2 stearate, PEG-6 stearate, PEG-6-32 stearate, PEG-8 stearate, PEG-9 stearate, PEG-20 stearate, PEG-40 stearate, PEG-45 stearate, PEG-50 stearate and PEG-100 stearate, and mixtures thereof.

In an embodiment, the non-ionic surfactant is a propylene glycol ester. Exemplary propylene glycol esters include, but are not limited to, propylene glycol laurate, propylene glycol palmitostearate, propylene glycol ricinoleate and propylene glycol stearate, and mixtures thereof.

In an embodiment, the non-ionic surfactant is a glyceryl ester or derivative. Exemplary glyceryl esters and derivatives include, but are not limited to, glyceryl behenate, glyceryl dibehenate, glyceryl dioleate, glyceryl distearate, glyceryl isostearate, glyceryl laurate, glyceryl linoleate, glyceryl monostearate, glyceryl oleate, glyceryl palmitate, glyceryl ricinoleate, glyceryl stearate, PEG-23 glyceryl cocoate, PEG-6 caprylic/capric glycerides, PEG-7 glyceryl cocoate, polyglyceryl-10 diisostearate, polyglyceryl-2 diisostearate, polyglyceryl-3 diisostearate and polyglyceryl-6 diisostearate, PEG-12 glyceryl laurate, PEG-120 glyceryl stearate, and mixtures thereof.

In an embodiment, the non-ionic surfactant is a polymeric ether. Exemplary polymeric ethers include, but are not limited to, poloxamer 124, poloxamer 181, poloxamer 182, poloxamer 184, poloxamer 188, poloxamer 237, poloxamer 331, poloxamer 338 and poloxamer 407, and mixtures thereof.

In an embodiment, the non-ionic surfactant is a sorbitan derivative. Exemplary sorbitan derivatives include, but are not limited to, polysorbate 20, polysorbate 40, polysorbate 60, polysorbate 65, polysorbate 80, sorbitan isostearate, sorbitan monolaurate, sorbitan monooleate, sorbitan monopalmitate, sorbitan monostearate, sorbitan sesquioleate, sorbitan trioleate and sorbitan tristearate, and mixtures thereof.

Another aspect of the invention is where the surfactant component comprises
a) at least one anionic surfactant which is an alkylpolyglucoside (AG) derivative surfactant selected from an alkylglycoside sulfonate, an alkylpolyglycoside sulfonate or a poly alkylpolyglycoside phosphate;
b) at least one non-ionic surfactant; and
c) at least one amphoteric surfactant.

In an embodiment the non-ionic surfactant is decyl glucoside (Plantaren 2000 NUP) or cocoglycoside (Pantacare 818UP). In one embodiment, the non-ionic surfactant is decyl glucoside. In another embodiment, the non-ionic surfactant is cocoglycoside. In another embodiment the non-ionic surfactant is a mixture of decyl glucoside and cocoglucoside. In one embodiment the amphoteric surfactant is Cocamidopropyl PG-Dimonium Chloride Phosphate.

Another aspect of the invention is where the surfactant component comprises
a) at least one anionic surfactant which is an alkylpolyglucoside (AG) derivative surfactant selected from an alkylpolyglycoside sulfonate or a poly alkylpolyglycoside phosphate;
b) at least one second anionic surfactant;
c) at least one amphoteric surfactant.

In one embodiment, the second anionic surfactant is sodium C14-16 Olefin Sulfonate. In another embodiment, the amphoteric surfactant is Cocamidopropyl PG-Dimonium Chloride Phosphate.

In another embodiment, the AG surfactant is Sodium Hydroxypropylphosphate Laurylglucoside Crosspolymer.

Suitably, in one embodiment the surfactant component for use herein includes and AG derivative surfactant, at least one anionic surfactant which is not an AG derivative, at least one non-ionic surfactant and optionally at least one amphoteric surfactant.

### Dermatologically acceptable excipients

The present topical pharmaceutical emulsion compositions comprise one or more additional dermatologically acceptable excipients. Suitably, the additional dermatologically acceptable excipient in includes, but is not limited to at least one of a pH adjusting agent, a chelating agent, a preservative, a coemulsifier, a co-solvent, a penetration enhancer, a humectant, a thickening or gelling or viscosity building agent, a fragrance, a colorant, and mixtures thereof.

### Humectants

The compositions of the invention comprise at least one humectant. Exemplary additional humectants include, but are not limited to glycerol, betaine, sarcosine, propylene glycol, butylene glycol, pentylene glycol, hexylene glycol, caprylyl glycol, sorbitol, glucose, other sugar alcohols and combinations or mixtures thereof. In an embodiment the humectant is glycerol. In another embodiment there is a mixture of humectants, e.g. glycerol and sorbitol.

Suitably, the at least one humectant is present in an amount from about 0.5 % to about 25% by weight, based on the total weight of the composition. In one embodiment, the at least one humectant is present in an amount from about 0.5 % to about 20% by weight, based on the total weight of the composition. Glycerol, if present, is suitably in an amount of about 1% to about 10 % w/w. In one embodiment, glycerine may be present in an amount from about 1 to about 5%. In one embodiment, glycerol is present in an amount of about 5% w/w. The term 'glycerin' and 'glycerol' is used interchangeably herein. Sorbitol if present is present in an amount of about 5 to about 20% w/w. In an embodiment, sorbitol is present from about 7.5 to about 15% w/w. Surprisingly it has been found that when the humectant is sorbitol, used in combination with the surfactant component system described herein, provides a BPO stabilizing role in the wash formulations of the present invention.

### Thickening Agent

The present topical pharmaceutical compositions may further comprise a thickening or gelling agent or rheology modifier. In an embodiment, the thickening agent is a mixture of two or more thickening agents.

The agent(s) and/or pH-independent agent(s) present in the gel or suspension have the role of increasing the viscosity of the aqueous phase. This makes it possible especially to improve the stabilization of this phase and its binding nature. In an embodiment, the gelling agent is a mixture of two or more gelling agents.

Exemplary thickening or gelling agents include, but are not limited to, agar, alginates such as the sodium alginate, arabinoxylan, carrageenan, celluloses and derivatives thereof, such as carboxymethylcellulose, hydroxyethylcellulose, hydroxypropylcellulose, hydroxypropyl methylcellulose, cellulose, curdlan, gelatin, gellan, β-glucan, tragacanth gum, gum arabic, pectin, starch, modified starches, such as the modified potato, a carbomer or a salt thereof, acrylate copolymers, silica, polysaccharides (such as xanthan or xanthan gum derivative such as dehydroxanthan gum & salts thereof, guar gum, locust bean gum, gum tragacanth and extracts of quince seeds), or a combination or mixture thereof.

Exemplary nonaqueous thickening agents include, but are not limited to, acrylate copolymers, VP/Eicosene copolymer, waxes, fatty alcohols and fatty acids.

Some carbomers and acrylates tend to be considered "electrolyte-insensitive" and are marketed by a large number of companies. Non limiting examples include Carbopol Ultrez- 20, Carbopol 1382 or Carbopol ETD 2020 by the company Noveon. Other carbomers include Carbopol 940 or 980 NF polymer (also referred to herein by the INCI name Carbomer Homopolymer).

In another embodiment, the acrylate copolymer is acrylates/C10-30 alkyl crosspolymer sold under the name Pemulen TR-I or Pemulen TR-2 by the company Noveon and the polyacrylate crosspolymer-6 (Sepimax Zen®). In one embodiment the gelling agent is a combination of at least two agents. In one embodiment the combination is a carbomer, such as Carbopol 980 and acrylate copolymer, such as polyacrylate crosspolymer-6.

Suitably, the gelling agent is present in the composition in an amount from about 0.1% to about 2.5% by weight. In one embodiment, the gelling agent is present in the composition in an amount from about 0.5% to about 2.5% by weight. In another embodiment the two polymers are suitable in about a 1:1 ratio to about a 1:4 ratio to each other. In one embodiment, the carbomer is higher, and in another embodiment the acrylate is higher.

Applicant's data suggests that a system with a combination of a carbomer and an acrylate copolymer produces physically stable formulations with a minimum pH shift, and with optimal viscosity minimally affected by BPO degradation and benzoic acid generation over time.

The reference to a stable formulation over time is to the physical stability of the formulation. This can be seen by there being no phase separation or settling. The BPO particles will remain as a suspension in the formulation. If the formulations are not properly stabilized by an appropriate gelling/viscosity building agent, then the suspension would settle out over time. Unexpectantly, the combination of the carbomer and an acrylate copolymer produces suspensions that do not settle under these conditions.

This is coupled with the minimization of a drop in the pH of the formulation over time. As used herein, a minimum pH shift refers to a drop in pH over time as the result of BPO degradation and conversion to benzoic acid. As noted previously, this is a common issue with BPO containing formulations due to degradation of the BPO and its conversion to benzoic acid. Unexpectantly, the combination of carbomer and an acrylate copolymer provides for a formulation with good buffering capability.

Suitably, a topical product ideally has a pH of between 4.5 and 5.5. It is desirable that the product not go below pH 4 as it's to acidic and ideally not above a pH of 8 0. The shift over time is relative to its shelf life. So, a minimum shift (drop of pH in this instance) at 6 mo. and at 30 C would be a less than 0.5 unit drop in pH at accelerated condition (30 C) over 6 months. In one embodiment, there is a less than 1 unit drop in pH at accelerated condition (30 C) over 6 months. In one embodiment, there is a less than 1.5 unit drop in pH at accelerated condition (30 C) over 6 months. This is in comparison to a the drop in pH from a product having a release pH of about 5.5.

The resulting formulation comprise at least one anionic surfactant which is a alkyl glucoside (AG) derivative surfactant selected from an alkylglycoside sulfonate, an alkylpolyglycoside sulfonate, or an alkylpolyglycoside phosphate; at least one humectant and at least one dermatologically acceptable excipient. In one embodiment the thickening agents are present in the composition in an amount from about 0.1% to about 2.5% by weight. In one embodiment, the thickening agents are present in the composition in an amount from about 0.5% to about 2.5% by weight. In another embodiment the thickening agent polymers are suitably present in about a 1:1 ratio to about a 1:4 ratio to each other.

In one aspect of the invention the pH values of the BPO formulation decreases less over time with the combination of thickening agents than a formulation without said thickening agents. One aspect of the invention is a decrease of 0.3 pH units after 2 months storage of the formulation at 25°C/60% RH. Another aspect is 0.2 pH units after 2 months storage of the formulation. In another aspect of the invention there is a decrease of 0.4 pH units after 2 months at accelerated storage conditions. In another aspect there is a decrease of 0.3 pH units after 2 months.

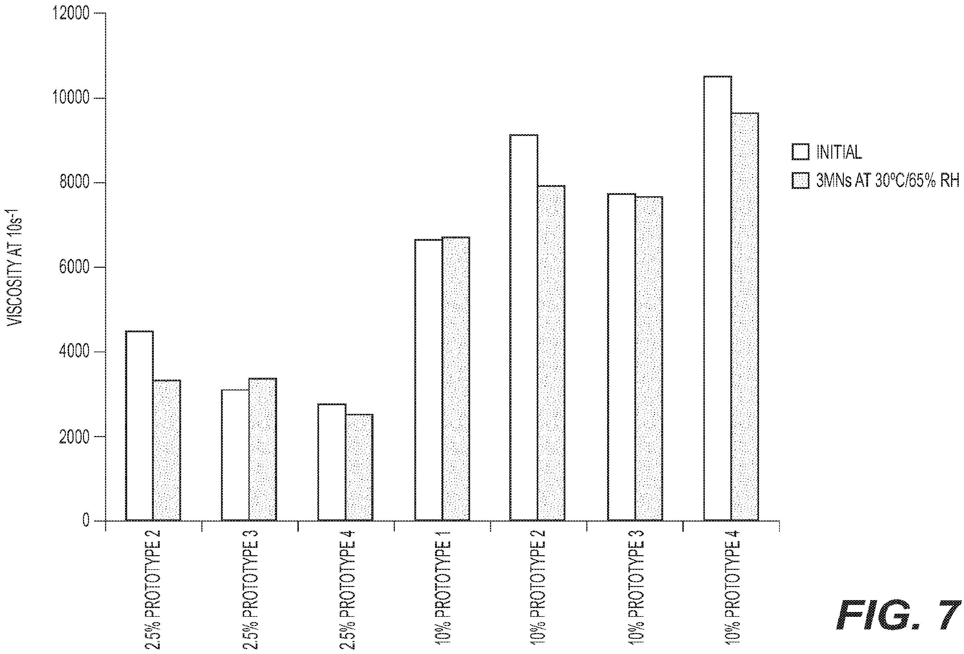

As shown in Figure 7 viscosity would also affect physical stability of the formulation. In extreme cases, a significant loss of viscosity in a suspension such as those herein would result in precipitation of the particles. Viscosity also affects sensory profiles of the formulation.

### Co-emulisifier

A fatty acid may be used as a co-emulsifying agent to assist in product texture as well as cleansing efficiency. In one embodiment, the thickening agent is a fatty acid which may be saturated or unsaturated, branched or straight chained), or a source of fatty acids, and mixtures thereof.

Exemplary fatty acids include, but are not limited to, isostearic acid, linoleic acid, linolenic acid, oleic acid, myristic acid, ricinoleic acid, columbinic acid, arachidic acid, arachidonic acid, lignoceric acid, nervonic acid, eicosapentanoic acid, palmitic acid, stearic acid and behenic acid, and mixtures thereof.

Other exemplary fatty acids include, but are not limited to lauric acid, tridecylic acid, myristic acid, pentadecylic acid, margaric acid, oleic acid, nonadecylic acid, arachidic acid, arachidonic acid, heneicosylic acid (C21), behenic acid (C22), tricosylic acid (C23), lignoceric acid (C24), pentacosylic acid(C25), cerotic acid (C26), heptacosylic acid (C27), montanic acid (C28), nonacosylic acid (C29), melissic acid (C30), henatriacontylic acid (C31), lacceroic acid (C32), psyllic acid (C33), geddic acid(C34), ceroplastic acid (C35) and hexatriacontylic acid (C36), and mixtures thereof.

The fatty acid can be introduced into the present compositions from a variety of sources. In an embodiment, the fatty acid is provided in the composition as an oil or wax. Examples of oils useful in this regard include, but are not limited to, rice bran oil, flaxseed oil, hempseed oil, pumpkin seed oil, canola oil, soybean oil, wheat germ oil, olive oil, grapeseed oil, borage oil, evening primrose oil, black currant seed oil, chestnut oil, corn oil, safflower oil, sunflower oil, sunflower seed oil, cottonseed oil, peanut oil, sesame oil and olus (vegetable) oil, including hydrogenated and non-hydrogenated versions thereof and mixtures thereof.

In one embodiment, the source of fatty acids is shea butter, also known as Butyrospermum parkii, if chemically treated. Shea butter comprises five principal fatty acids, namely palmitic acid, stearic acid, oleic acid, linoleic acid and arachidic acid. Shea butter also comprises phytosterols.

In one embodiment the fatty acid is stearic acid. The fatty acid is suitable present in an amount of from about 0.1 to about 4.0 % w/w, based upon the total weight of the composition.

In another embodiment, the fatty acid is present in an amount of about 0.5 to about 2.5 % w/w. In one embodiment the stearic acid is present in an about of about 2% w/w.

In an embodiment, the coemulsifier is a fatty alcohol which may be saturated or unsaturated, branched or straight chained. Exemplary fatty alcohols include, but are not limited to, isostearyl alcohol, caprylyl alcohol, decyl alcohol, lauryl alcohol, myristyl alcohol, behenyl alcohol, lanolin alcohol, arachidyl alcohol, oleyl alcohol, palm alcohol, isocetyl alcohol, cetyl alcohol, stearyl alcohol and cetearyl alcohol, and mixtures thereof. In one embodiment, the fatty alcohol is a mixture of cetyl alcohol and stearyl alcohol, known as cetearyl alcohol (which is also known as cetostearyl alcohol).

Other suitable fatty alcohols include, but are not limited to, tridecyl alcohol, pentadecyl alcohol, isocetyl alcohol, palmitoleyl alcohol, heptadecyl alcohol, isostearyl alcohol, oleyl alcohol, nonadecyl alcohol, heneicosyl alcohol, erucyl alcohol, lignoceryl alcohol, ceryl alcohol, 1-heptacosanol, montanyl alcohol, 1-nonacosanol, myricyl alcohol, lacceryl alcohol, geddyl alcohol, tetratriacontanol, and lanolin alcohol, and mixtures thereof.

The fatty alcohol is suitably present in an amount of from about 0.1 to about 4.0% w/w, based upon the total weight of the composition. In another embodiment, the fatty alcohol is present in an amount of about 0.5 to about 2.5 % w/w.

### pH adjusting agent

The compositions of the invention may further comprise a pH adjusting agent. In one embodiment, the pH adjusting agent is a base. Suitable bases include amines, bicarbonates, carbonates, and hydroxides such as alkali or alkaline earth metal hydroxides, as well as transition metal hydroxides. In an embodiment, the base is sodium hydroxide or potassium hydroxide.. In an embodiment, the base is sodium hydroxide.

Suitably, the pH adjusting agent is present in the composition in an amount from about 0.01% to about 10% by weight. In an embodiment, the pH of the composition is adjusted with a pH adjusting agent to a pH of from about 4 to about 7, such as from about 4.5 to about 6.5. In one embodiment the pH is about 5.0 ± 0.2.

### Preservatives

While not believed to be necessary, the present topical pharmaceutical emulsion compositions may further comprise a preservative if desired. In an embodiment, the preservative is a mixture of two or more preservatives.

Exemplary preservatives include, but are not limited to, benzyl alcohol, imidazolidinyl urea, diazolidinyl urea, dichlorobenzyl alcohol, chloroxylenol, methyl paraben, ethyl paraben, propyl paraben, butyl paraben, phenoxyethanol, sorbic acid, benzoic acid, salts thereof, and mixtures thereof.

### Chelating agents

The present topical pharmaceutical emulsion compositions may further comprise a chelating agent. In an embodiment, the chelating agent is a mixture of two or more chelating agents. As described herein, the compositions of the invention may comprise a mixture of a chelating agent and an antioxidant, where both excipients act to prevent or minimize oxidative degradation reactions in the composition.

Exemplary chelating agents include, but are not limited to, citric acid, glucuronic acid, sodium hexametaphosphate, zinc hexametaphosphate, ethylene diamine tetraacetic acid (EDTA), phosphonates, salts thereof, and mixtures thereof. Ethylene diamine tetraacetic acid is also known as edetic acid. In one embodiment, the chelating agent is EDTA or a salt thereof, such as potassium, sodium or calcium salts of EDTA. In an embodiment, the EDTA or a salt thereof is disodium EDTA.

Suitably, the chelating agent is present in the composition in an amount from about 0.01% to about 1% by weight, based on the total weight of the composition. In one embodiment, the chelating agent is present in the composition in an amount of about 0.1% by weight, based on the total weight of the composition.

In one embodiment of the disclosure, there is provided a topical pharmaceutical or cosmetic cleanser composition comprising
a) benzoyl peroxide present in an amount from about 2.0 to about 20% w/w;
b) water,;
c) at least one anionic surfactant which is an alkylglucoside (AG) derivative surfactant selected from an alkylglycoside sulfonate, an alkylpolyglycoside sulfonate, or an alkylpolyglycoside phosphate;
d) at least one non-ionic surfactant;
e) at least one humectant;
f) a thickening agent; and
g) optionally at least one dermatologically acceptable excipient.

This topical cleanser composition is useful for the treatment of acne.

In one embodiment, the amount of BPO present is from about 2.5 to about 10% w/w. In one embodiment, the amount of BPO present is from about 4.0 to about 10% w/w. In one embodiment, the amount of BPO present is about 4.0, and in another embodiment the amount of BPO present is about 10% w/w. In yet another embodiment, the amount of BPO present is about 2.0%, 2.5%, 3 %, 4%, 5%, 6%, 7%, 8%, 9% and 10% w/w.

Suitably, the at least one humectant is present in an amount from about 0.5 % to about 20% by weight, based on the total weight of the composition. Glycerol, if present, is suitably in an amount of about 1% to about 10% w/w. Sorbitol, if present is present in an amount of about 5 to about 20% w/w. In an embodiment, sorbitol is present from about 7.5 to about 15% w/w. In one embodiment there is a mixture of humectants, e.g. glycerol and sorbitol.

In one embodiment of the disclosure, there is provided a topical pharmaceutical or cosmetic cleanser composition comprising
a) benzoyl peroxide present in an amount from about 2.0 to about 20% w/w;
b) water,
c) at least one anionic surfactant which is an alkylglucoside (AG) derivative surfactant selected from an alkylglycoside sulfonate, an alkylpolyglycoside sulfonate, or an alkylpolyglycoside phosphate;
d) at least one non-ionic surfactant;
e) at least one humectant present in an amount of from about 5 to about 25% w/w;
f) a thickening agent, and
g) optionally at least one dermatologically acceptable excipient.

In one embodiment of the disclosure, there is provided a topical pharmaceutical or cosmetic cleanser composition comprising
a) benzoyl peroxide present in an amount from about 2.0 to about 20% w/w;
b) water,
c) at least one anionic surfactant which is an alkylglucoside (AG) derivative surfactant selected from an alkylglycoside sulfonate, an alkylpolyglycoside sulfonate, or an alkylpolyglycoside phosphate;
d) at least one non-ionic surfactant;
e) at least one humectant present in an amount of from about 5 to about 25% w/w;
f) a thickening agent present in an amount of about 0.1% to about 2.5%; and
g) optionally at least one dermatologically acceptable excipient.

In one embodiment, the at least one dermatologically acceptable excipient is selected from a co-emulsifier, a pH adjusting agent, at least one preservative, a chelating agent, and a combination or mixture thereof.

In one embodiment of the disclosure, there is provided a topical pharmaceutical or cosmetic cleanser composition comprising
a) benzoyl peroxide present in an amount from about 2.0 to about 20% w/w;
b) water,
c) at least one anionic surfactant which is an alkylglucoside (AG) derivative surfactant selected from an alkylglycoside sulfonate, an alkylpolyglycoside sulfonate, or an alkylpolyglycoside phosphate;
d) at least one non-ionic surfactant;
e) at least one humectant present in an amount of from about 5 to about 25% w/w;
f) a thickening agent selected from an acrylate or carbomer, or a combination or mixture thereof; and
g) optionally at least one dermatologically acceptable excipient.

In one embodiment, the carbomer is Carbomer Homopolymer. In another embodiment, the acrylate is Polyacrylate crosspolymer-6, and/or acrylates/C10-30 alkyl crosspolymer. In another embodiment, the acrylate is Polyacrylate crosspolymer-6.

In one embodiment of the disclosure, there is provided a topical pharmaceutical or cosmetic cleanser composition comprising
a) benzoyl peroxide present in an amount from about 2.0 to about 20% w/w;
b) water,
c) at least one anionic surfactant which is Sodium Laurylglucoside Hydroxypropyl sulfonate;
d) at least one non-ionic surfactant which is decyl glucoside or cocoglycoside, or a combination or mixture thereof;
e) at least one humectant present in an amount of from about 5 to about 25% w/w;
f) a thickening agent selected from an acrylate or carbomer, or a combination or mixture thereof; and
g) optionally at least one dermatologically acceptable excipient.

In one embodiment the additional dermatologically acceptable excipient is selected from a pH adjusting agent, co-emulsifier, a chelating agent, a preservative, a co-solvent, a penetration enhancer, a fragrance, a colorant, and mixtures thereof.

In an embodiment the co-emulsifier is a fatty acid. In an embodiment the fatty acid is behenic acid or stearic acid, or a combination or mixture thereof.

In an embodiment the pH adjusting agent is sodium or potassium hydroxide.

### Methods of Preparation

A formulation of the present invention may be prepared as follows:
In a glass beaker, Carbopol 980 NF is hydrated in water. The gel is heated to 60°C while agitating with an overhead mixer (500 rpm). Sepimax Zen is then added to the gelled mixture slowly. The gel is mixed until complete polymer hydration occurs. In a separate beaker, glycerin, surfactants and stearic acid are hand mixed and heated to 70 °C. The mixture is then added to the main beaker under agitation (1200-1800 rpm). The formulation is cooled down to below 30 °C while mixing and the BPO source, such as Curoxyl 42 is then added. If required, the final pH is adjusted to 5.00 ± 0.20 using 10% NaOH.

### Evaluation of the BPO stability

BPO stability in the formulations of the present invention were assessed over time at 25°C/60% RH and 30°C/65% RH using environmentally controlled chambers. Specifically formulations containing 2.5% BPO or 10% BPO in glass vials were placed on stability, and monitored for BPO degradation.

Physical stability of the formulations was assessed using a Centrifuge test. The samples were placed in 15 ml centrifuge tubes and were spun at 2250 g for 30 minutes. A "PASS" score was assigned if less than 0.05% of the BPO particles settled on visual inspection.

Stability samples were assessed visually for appearance and pH, e.g. phase separation and color change. The pH was also measured using a solid pH probe at each stability time point.

BPO stability is significantly affected by the surfactants chemical composition. Specifically, surfactants with primary and secondary amine structures have a catastrophic effect on BPO stability. For example, surfactants such as Cola lipid C, Cola Teric CDCX-50, and ChemBetain C cause significant BPO degradation in binary mixtures. This is in agreement with the published observations of BPO solutions in aniline, triethylamine, etc. being explosive (K. Nozaki et al., Inth J. Am Chem Soc., 68:1686-1692 (1946).

It has also been found that when amphoteric surfactants such as Cola Lipid C become complexed to an anionic surfactant, such as Colonial AOS, it does not interact with BPO to the same extent as without the anionic surfactant being present. Surprisingly it has been found that the AG sugar based surfactants and their derivatives, such as those described herein, result in the least amount of BPO degradation. This makes their use in a formulation of BPO, including low dose BPO formulations, unexpectantly successful in producing a product which will meet EP and other countries more stringent standards.

BPO's solubility in water is 0.003 mg/ml. The addition of surfactants will significantly increase BPO solubility. The increase in solubility of the BPO is dependent on the surfactant type. It is expected that increasing BPO solubility will decrease its stability. Once in solution the BPO decomposes quickly in water, with the half-life of 11.87 hours at pH 4 and 5.20 hours at pH 7. The anionic AG surfactants and their derivatives, such as PolySuga Glycinate increased the BPO solubility the least, e.g. to 0.058 mg/ml.

Applicants have found that BPO solubility is correlated with its stability. However, Applicants have found that the correlation is not linear, with BPO degrading much faster above solubility values of 0.25 mg/ml.

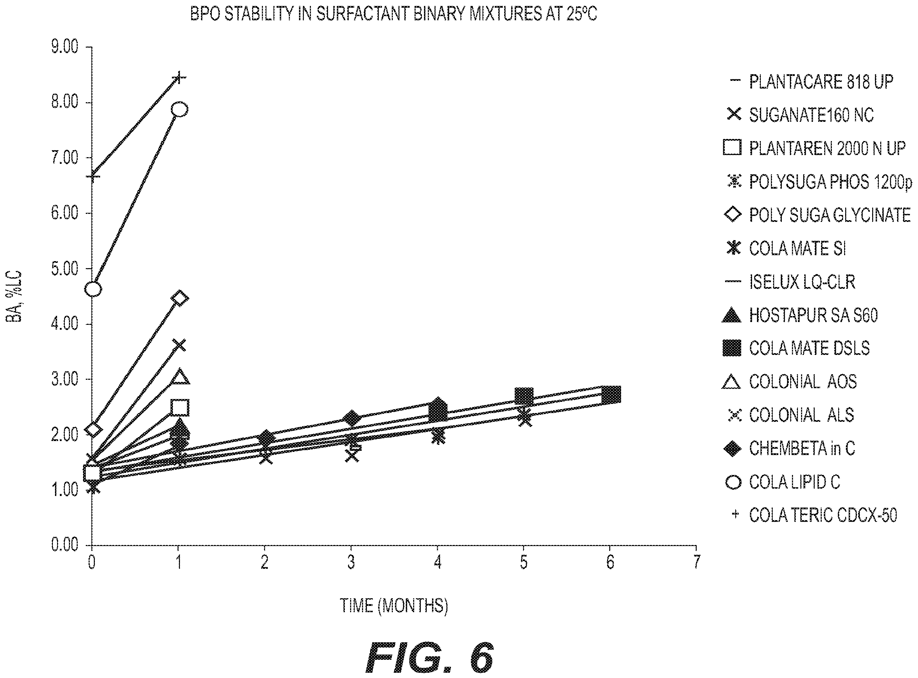

As is shown in Figure 6, the stability data of 2.5% BPO wash formulations with sugar based surfactants (formulation #4) as compared to non-sugar based surfactant containing formulations (formulations #22 and #23) is superior.

As shown in Figure 6, the stability of Curoxyl 42 (BPO) as demonstrated by BPO degradation in binary mixtures of various surfactants is unexpectantly improved when combined with sugar based surfactants. The concentration of all surfactants was fixed at 6% w/w active. As can be seen, addition of a surfactant significantly increases the solubility of BPO. The increase in solubility appears to be dependent on the surfactant type. Surfactants such as Cola lipid C, Cola Teric CDCX-50 and ChemBetain C cause significant BPO degradation in binary mixtures. PolySuga Glycinate increased BPO solubility to 0.058 mg/ml while Hostapon CT paste increased it to 0.481 mg/ml. It is expected that increasing BPO solubility would decrease its stability as once in solution, BPO decomposes quickly, e.g. in water - a half-life of 11.87 hours at pH 4 and 5.20 hours at pH 7.

Applicants have further demonstrated that BPO solubility is correlated with its stability.

In one embodiment, the composition may optionally comprise at least one dermatologically acceptable excipient. The at least one dermatologically acceptable excipient is selected from a co-emulsifier, a pH adjusting agent, at least one preservative, a chelating agent, a co-solvent, a penetration enhancer, a fragrance, a colorant, or a combination or mixture thereof.

### Definitions

The phrase "therapeutically effective amount" is used herein to refer to an amount sufficient to have a therapeutic effect upon administration. Effective amounts will vary with the particular condition being treated, the severity of the condition, the duration of the treatment, and the specific components of the composition.

The terms "administering" and "administration" are used herein to mean any method which in sound medical practice delivers the pharmaceutical composition to a patient in such a manner as to provide the desired therapeutic effect.

As used herein, "topical" administration of the composition refers to application of the composition to the stratum corneum.

The terms "treatment" or "treating" need not mean that the condition or disorder is totally cured. A useful pharmaceutical composition herein need only to reduce the severity of the condition or disorder, reduce the severity of symptoms associated therewith, provide improvement to a patient's quality of life, or delay, prevent or inhibit the onset of the condition or disorder. A treatment need not be effective in every member of a population to have clinical utility, as is recognized in the medical and pharmaceutical arts.

The term "hyper cornification" as associated with acne is considered one of the causes for development of acne lesions and sometimes the definition of hypercornification or (ductal hypercornification) can be seen in this process. Sebum flows through the canal of the sebaceous follicle where there is an increase in the production of corneocytes lining the follicles and an abnormal increase with excess sebum flow forming a bulging mass called microcomedones.

As used herein, "Folliculitis" is the term given to a group of skin conditions in which there are inflamed hair follicles. Acne and its variants are types of folliculitis.

The term "acne" as used herein mean a follicular disorder that may encompass both non-inflammatory lesions (e.g., open comedones (blackheads); closed comedones (whiteheads); cysts; and inflammatory lesions (e.g. papules, pustules and inflammatory nodules). There are also secondary lesions such as excoriations; postinflammatory erythematous macules; postinflammatory pigmented macules (in dark skin); and scars.

The term "pharmaceutically acceptable" means approvable by a regulatory agency or listed in a Pharmacopeia or other generally recognized guide for use in animals, and more particularly in humans. As used herein "pharmaceutically acceptable" is applicable to monograph, OTC or other consumer product usage.

As used herein, the term "skin penetration" refers to the diffusion of the active agent through the stratum corneum and into the epidermis and/or dermis of the skin or the systemic circulation.

As used herein, "patients" includes human patients. Included within human patients are adults, children and adolescents.

As used herein, "substantially free" of a specified component refers to a composition with less than about 1% by weight of the specified component. "Free" of a specified component refers to a composition where the specified component is absent.

Any concentration range, percentage range or ratio range recited herein is to be understood to include concentrations, percentages or ratios of any integer within that range and fractions thereof, such as one tenth and one hundredth of an integer, unless otherwise indicated. This interpretation should apply regardless of the breadth of the range or the characteristic being described.

Unless otherwise indicated, all percentages are based on the percentage by weight, e.g. w/w of the final composition prepared, and all totals equal 100% by weight.

It should be understood that the terms "a" and "an" as used herein refer to "one or more" or "at least one" of the recited components. It will be clear to one of ordinary skill in the art that the use of the singular includes the plural unless specifically stated otherwise.

Throughout the specification, descriptions of various embodiments use "comprising" language, however in some specific instances, an embodiment can alternatively be described using the language "consisting essentially of' or "consisting of'.

All numbers expressing quantities, percentages or proportions, and other numerical values used in the specification, are to be understood as being modified in all instances by the term "about".

As used herein the term "long chain" or "fatty" such as used in reference to "fatty alcohol" or "fatty acid", etc. refers to a hydrocarbon backbone chain which may be straight or branched, saturated or unsaturated, and is suitably composed of 12 to 36 carbon atoms. In one embodiment, the chain is 12 to 30. In another embodiment, it is 16 to 26 carbon atoms. In another embodiment the chain is 16 to 22 carbon atoms. In one embodiment, the chain is 22 to 30 carbon atoms. In one embodiment, the chain is 16 to 26 carbon atoms. In another embodiment the chain is 16 to 22 carbon atoms. In another embodiment, the chain is 20 to 22 carbon atoms. In another embodiment, the chain is from 20 to 30 carbon atoms, suitably 22 to 30 carbon atoms. In another embodiment the chain is from 22 to 28 carbon atoms.

Other terms used herein are intended to be defined by their well-known meanings in the art.

### BIOLOGICAL EXAMPLES

### Evaluation of the skin irritation potential of individual surfactants and placebo formulations

Irritancy potential of the individual surfactants as well as formulation chassis (placebo formulations) were evaluated using an in house *in vitro* test. The method utilizes HaCaT cell viability as a marker for irritation. The data have been shown to be reproducible.

Briefly, HaCaT cells were seeded in 96- well plates (Nunc 96 well plate, Cat# 165305) at density of 2 x 10⁴ cells per well, and grown overnight before treatments. Test articles were diluted in water to make 10% stock solutions, which were serially diluted in DMEM to twice to the final treatment concentrations. Cells were treated in triplicates by adding 100 µl of serially diluted test articles to each well containing 100 µl fresh medium. After incubation at 37°C, 5% CO₂ for 10 minutes in the presence of diluted placebos, cells were then washed with 200 µl of DPBS with Ca⁺⁺ and Mg⁺⁺ (Life Technologies # 14040133) for 3 times. CellTiter-Blue reagent (Promega, G8081, 10 x stock solution) was diluted to 1x working solution in cell culture medium and added to cells (100 µl/well). Cells were incubated at 37°C, 5% CO₂ for 60 minutes and the cell viability was measured using fluorescent activity (Ex. 560 nm, Em. 590 nm). All treated samples were normalized to the untreated control. Cell viabilities were then used to assess skin irritation potential.

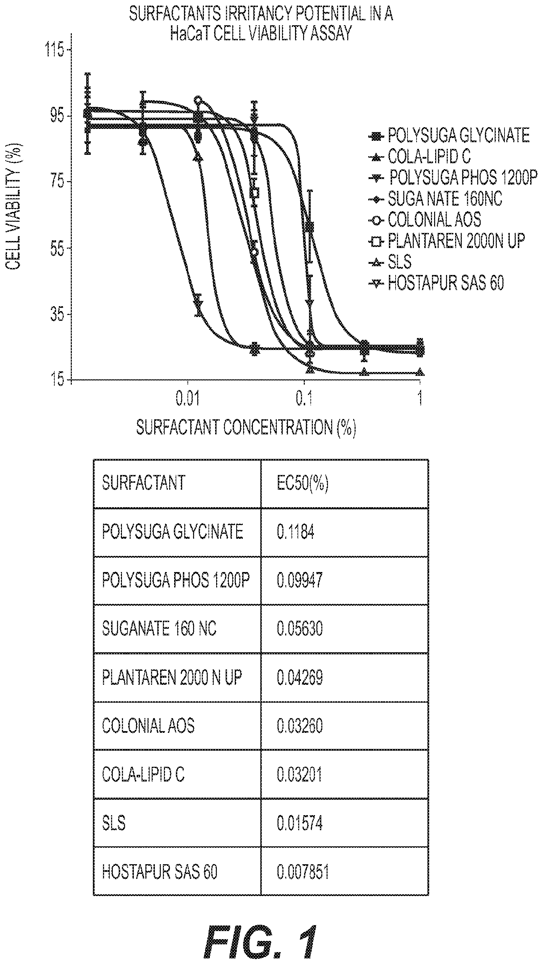

Figure 1 shows the irritancy potential of selected surfactants in a HaCaT Cell viability assay. SLS has been included as the positive control. Based on the EC 50 values, the sugar based surfactants are found to be extremely mild. The data is in line with the published data as well as the supplier data.

The screened surfactants rank as follow:
PolySuga Glycinate C > PolySuga Phos 1200P > Suganate 160 NC > Plantaren 2000 N UP > Colonial AOS > Cola Lipid C > SLS > Hostapur SAS 60.

Applicants have found that when looking at non-ionic surfactant concentration and sorbitol concentrations, viscosity decreases with increase in the surfactant decyl glucoside, while foaming increases with increasing the surfactant concentration. Sorbitol appears to have no effect on the foaming and minimum effect on viscosity. Most interestingly, sorbitol appears to improve BPO stability as less benzoic acid is generated with increasing sorbitol concentrations.

Tables 1-5 below describe exemplary formulations of the present invention based upon a 2.5% and 10% BPO concentration. The formulations contain a 5% processing overage. A target pH is 5.00 ± 0.20 to ensure BPO stability and pH balanced formulations for optimum skin mildness.

In tables 1-5 below, compositions #21, #22 et #23 are reference examples.

**Table 1 Compositions containing 2.5% Benzoyl Peroxide**

| **Ingredients** | | **# 1** | **#2** | **#3** | **#4** |
|---|---|---|---|---|---|
| **INCI Name** | **Trade Name** | wt.% | wt.% | wt.% | wt.% |
| Carbo mer | Carbopol 980 NF | 0.500 | 0.500 | 0.500 | 0.500 |
| Polyacrylate Copolymer-6 | Sepimax Zen | 0.800 | 0.500 | 0.500 | 0.500 |
| Benzoyl Peroxide¹ | Curoxyl 42 | 2.625 (6.604) | 2.625 (6.604) | 2.625 (6.604) | 2.625 (6.604) |
| Glycerol | Glycerin, USP | 5.000 | 5.000 | 5.000 | 5.000 |
| D-Sorbitol | Neosorb P 60W | 15.000 | 0.000 | 7.5000 | 15.000 |
| Stearic Acid | FA-1890V | 0.000 | 2.000 | 2.000 | 2.000 |
| Sodium Laurylglucoside Hydroxypropylsulfonate | Suganate 160 NC² | 8.00 (20.000) | 4.00 (10.000) | 4.00 (10.000) | 4.00 (10.000) |
| Decyl Glucoside | Plantaren 2000 N UP³ | 0.000 | 1.00 (2.000) | 1.50 (3.000) | 2.00 (4.000) |
| Sodium 4Hydroxide⁴ | Sodium Hydroxide | 0.075 | 0.086 | 0.075 | 0.054 |
| Water | Water | 52.02 | 73.31 | 64.82 | 56.34 |

| | | | | | |
|---|---|---|---|---|---|
| ¹ 6.604% Curoxyl 42 (2.5% BPO equivalent + 5% processing overage; ²Sodium Laurylglucoside Hydroxypropylsulfonate (Suganate 160 NC) contains 40% active, ³Decyl Glucoside (Plantarene 2000 N UP) contains 50% active; ⁴Target pH: 5.00 ± 0.20 | | | | | |

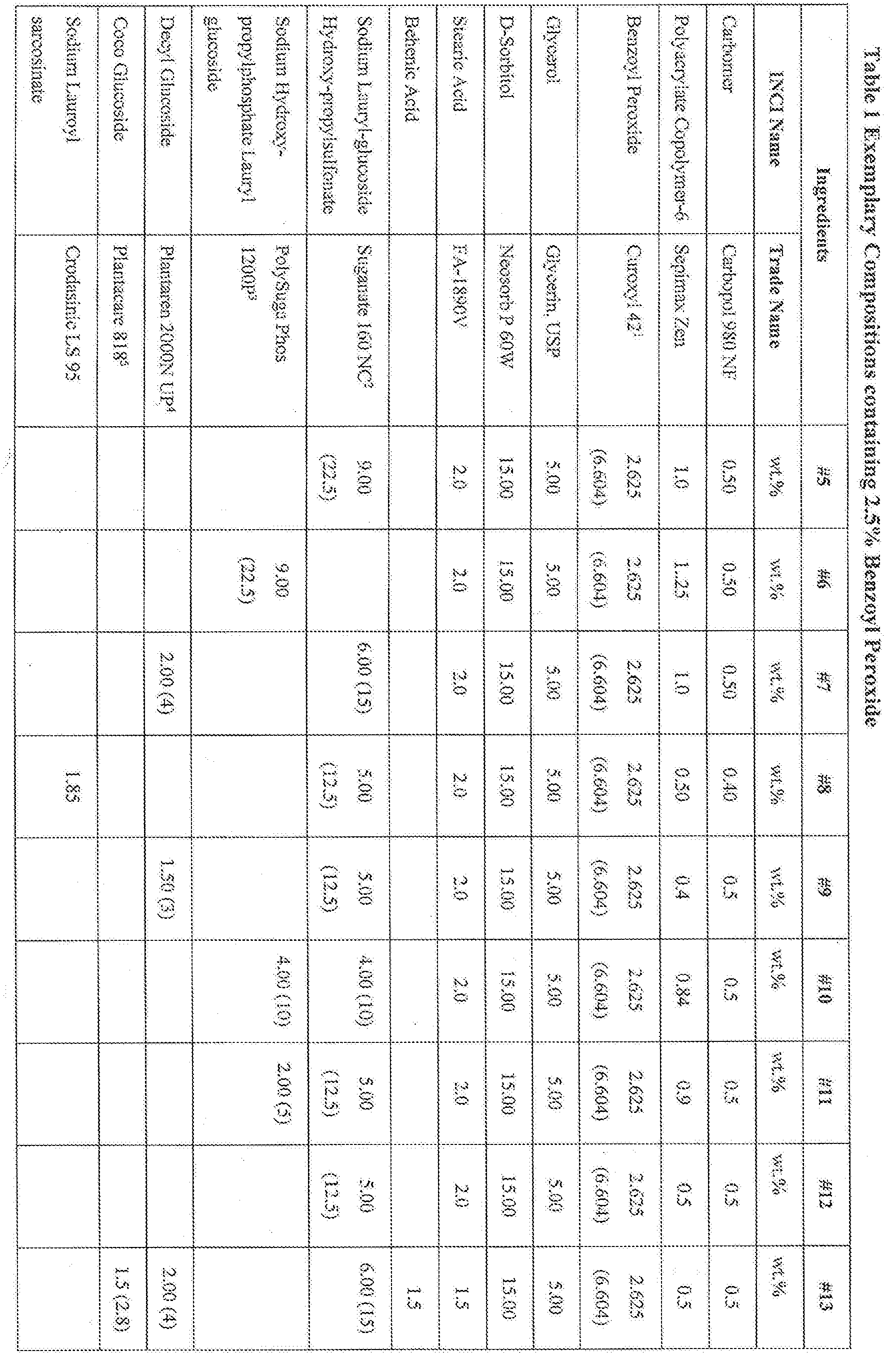

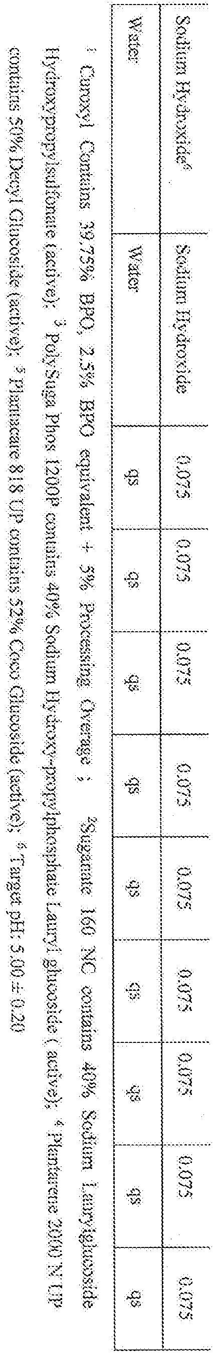

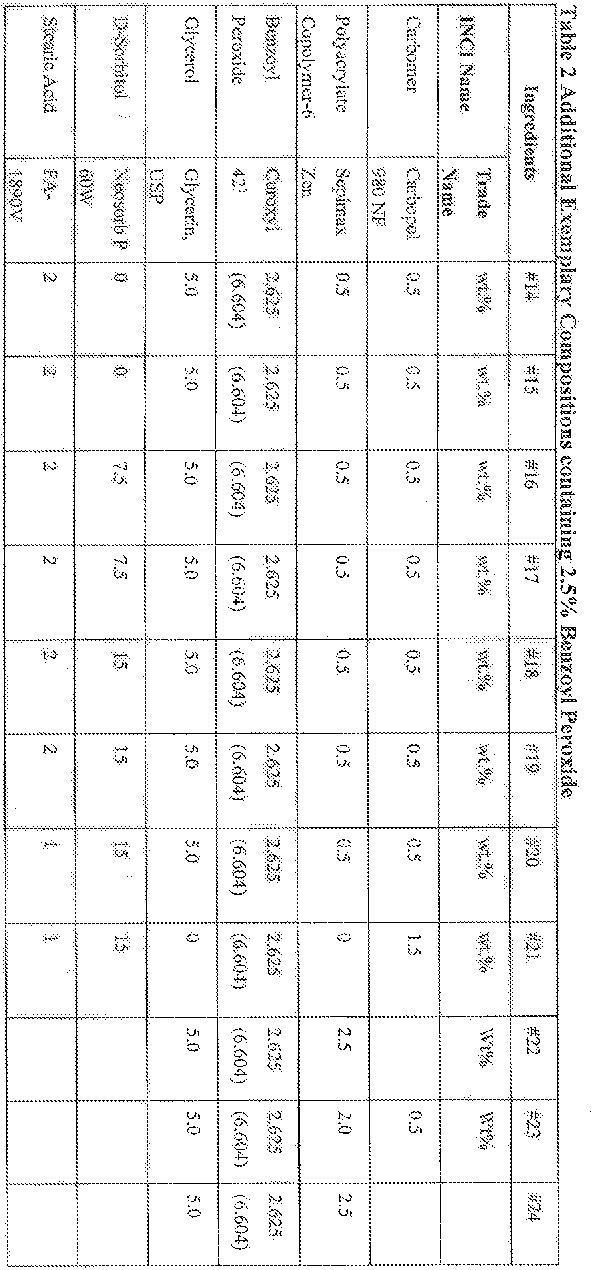

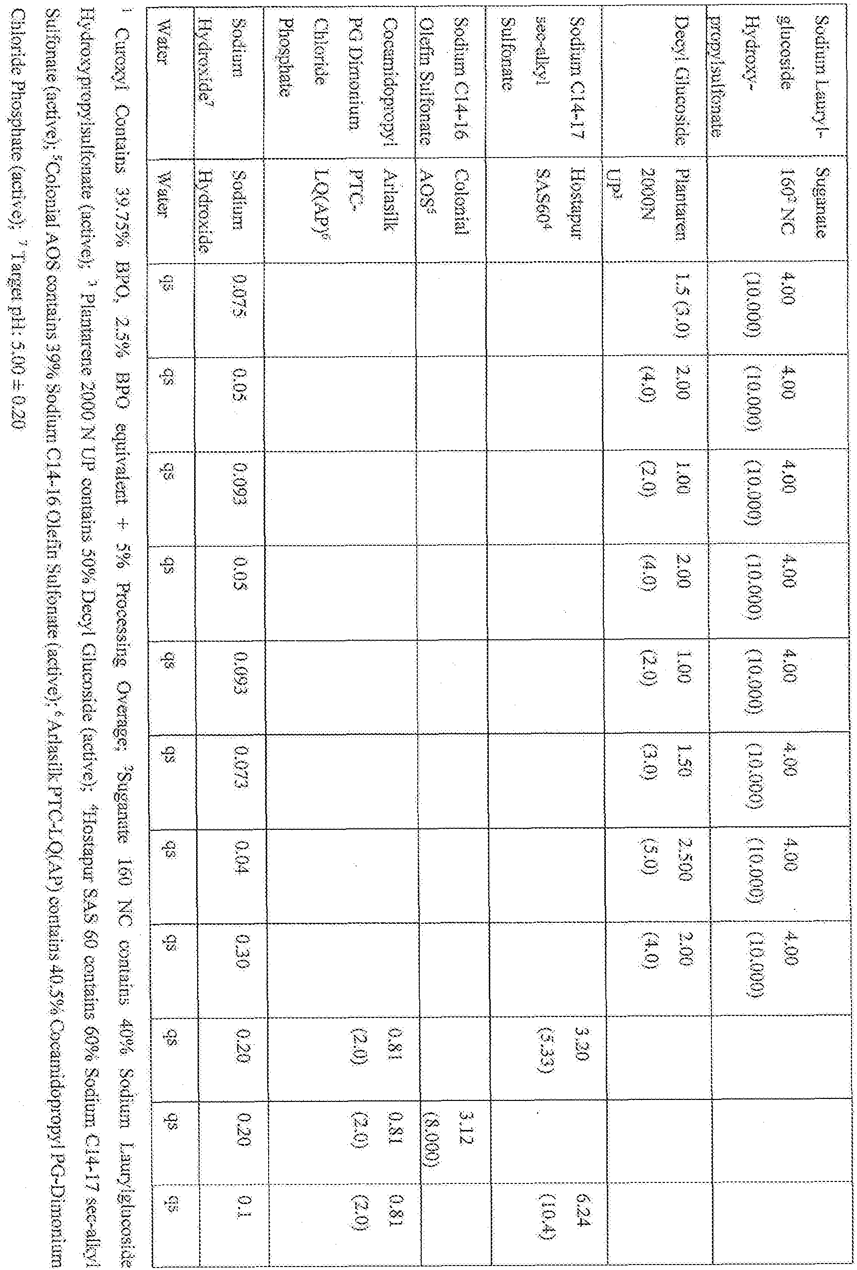

**Table 4 Compositions containing 10% Benzoyl Peroxide**

| **Ingredients** | | **# 1** | **# 2** | **# 3** | **# 4** |
|---|---|---|---|---|---|
| **INCI Name** | **Trade Name** | wt.% | wt.% | wt.% | wt.% |
| Carbomer | Carbopol 980 NF | 0.7000 | 0.7000 | 0.5000 | 0.7000 |
| Polyacrylate Copolymer-6 | Sepimax Zen | 0.8000 | 0.8000 | 1.800 | 0.8000 |
| Benzoyl Peroxide | Curoxyl 42¹ | 10.50 (26.415) | 10.50 (26.415) | 10.50 (26.415) | 10.50 (26.415) |
| Glycerol | Glycerin, USP | 5.000 | 5.000 | 5.000 | 5.000 |
| D-Sorbitol | Neosorb P 60W | 0.000 | 0.000 | 0.000 | 7.500 |
| Stearic Acid | FA-1890V | 2.000 | 2.000 | 0.000 | 2.000 |
| Sodium Lauryl-glucoside Hydroxy-propylsulfonate | Suganate 160 NC² | 10.50 (26.415) | 10.50 (26.415) | 10.50 (26.415) | 10.50 (26.415) |
| Decyl Glucoside | Plantaren 2000 N UP³ | 4.25 (9.500) | 4.25 (9.500) | 0.000 | 4.25 (9.500) |
| Coco-glycoside | Plantacare 818 UP⁴ | 0.000 | 1.82 (3.500) | 0.000 | 0.000 |
| Sodium C14-16 Olefin Sulfonate | Colonial AOS⁵ | 0.000 | 0.000 | 3.12 (8.000) | 0.000 |
| Cocamidopropyl PG-Dimonium Chloride Phosphate | Arlasilk PTC-LQ(AP)⁶ | 0.000 | 0.000 | 0.81 (2.000) | 0.000 |
| Sodium Hydroxide⁷ | Sodium Hydroxide | 0.000 | 0.000 | 0.065 | 0.000 |
| Water | Water | 45.59 | 52.09 | 56.22 | 38.09 |

| | | | | | |
|---|---|---|---|---|---|
| ¹ 10% BPO equivalent + 5% Processing Overage; ² Suganate 160 NC contains 40% Sodium Laurylglucoside Hydroxypropylsulfonate (active); ³ Plantarene 2000 N UP contains 50% Decyl Glucoside (active); ⁴ Plantacare 818 UP contains 52% Coco Glucoside (active); ⁵Colonial AOS contains 39% Sodium C14-16 Olefin Sulfonate (active); ⁶ Arlasilk PTC-LQ(AP) contains 40.5% Cocamidopropyl PG-Dimonium Chloride Phosphate (active); ⁷Target pH: 5.00 ± 0.2 | | | | | |

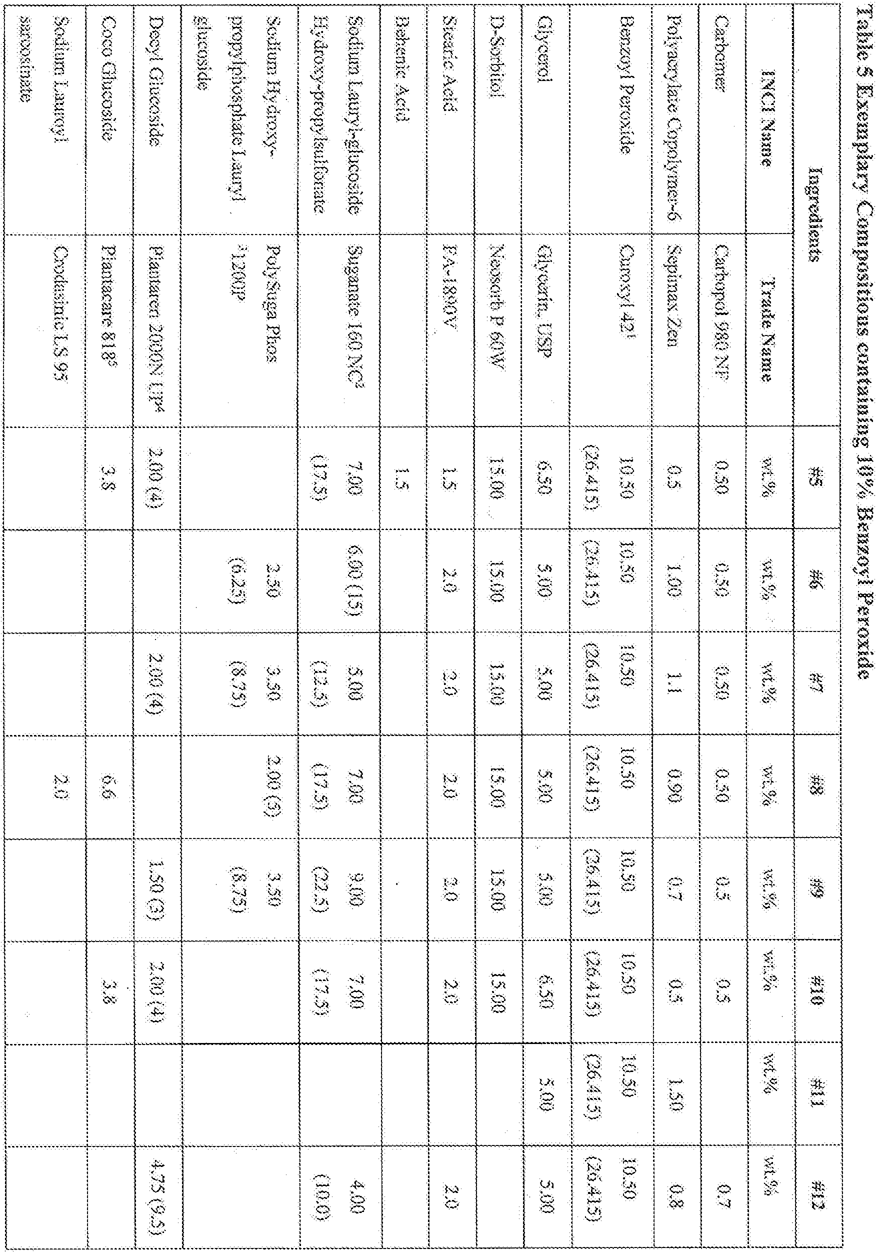

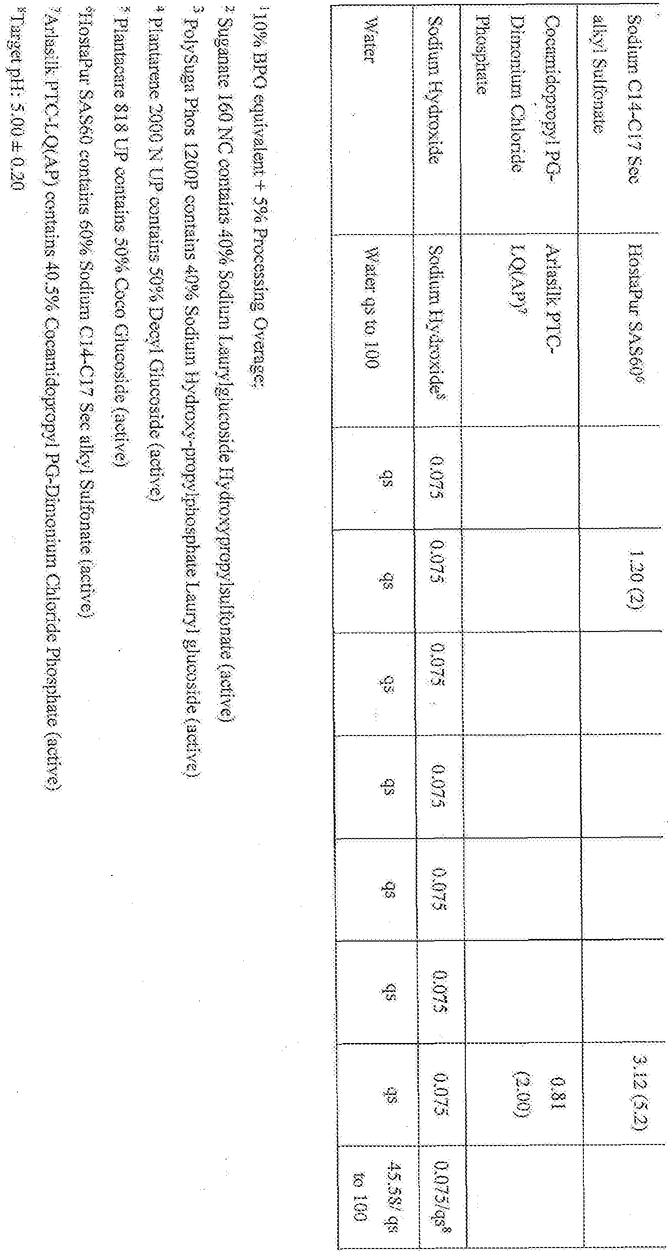

It should be noted that formulations containing 4.0% Benzoyl Peroxide can be simply made by adjusting the % w/w of BPO from those of the above noted 2.5% BPO or the 10% BPO formulations, and all of those variations are within the scope of this invention. Minor adjustments may also be necessary to the thickening agents and the sodium hydroxide, for example, but well within the skill of one in the art.

One example of a representative 4% BPO formulation is as shown below in Table 6:

**Table 6 Compositions containing 4.0% Benzoyl Peroxide**

| **Ingredients** | | **# 13** |
|---|---|---|
| **INCI Name** | **Trade Name** | **wt.%** |
| Carbo mer | Carbopol 980 NF | 0.600 |
| Polyacrylate Copolymer-6 | Sepimax Zen | 0.500 |
| Benzoyl Peroxide¹ | Curoxyl 42 | 4.4 (10.56) |
| Glycerol | Glycerin, USP | 5.000 |
| D-Sorbitol | Neosorb P 60W | 15.000 |
| Stearic Acid | FA-1890V | 2.000 |
| Sodium Laurylglucoside Hydroxypropylsulfonate | Suganate 160 NC² | 4.00 (10.000) |
| Decyl Glucoside | Plantaren 2000 N UP³ | 2.00 (4.000) |
| Sodium Hydroxide⁴ | Sodium Hydroxide | 0.07 |
| Water | Water | 51.76 |

| | | |
|---|---|---|
| ¹ 6.604% Curoxyl 42 (4.0% BPO equivalent + 10% processing overage; ²Sodium Laurylglucoside Hydroxypropylsulfonate (Suganate 160 NC) contains 40% active, ³Decyl Glucoside (Plantarene 2000 N UP) contains 50% active; ⁴Target pH: 5.00 ± 0.20 | | |

### Skin irritation potential of the chassis

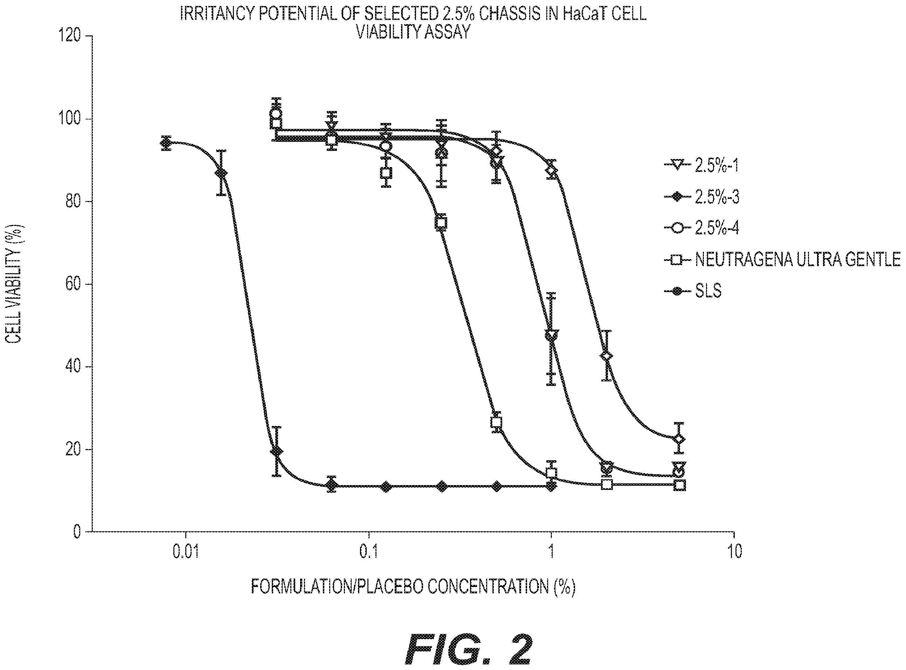

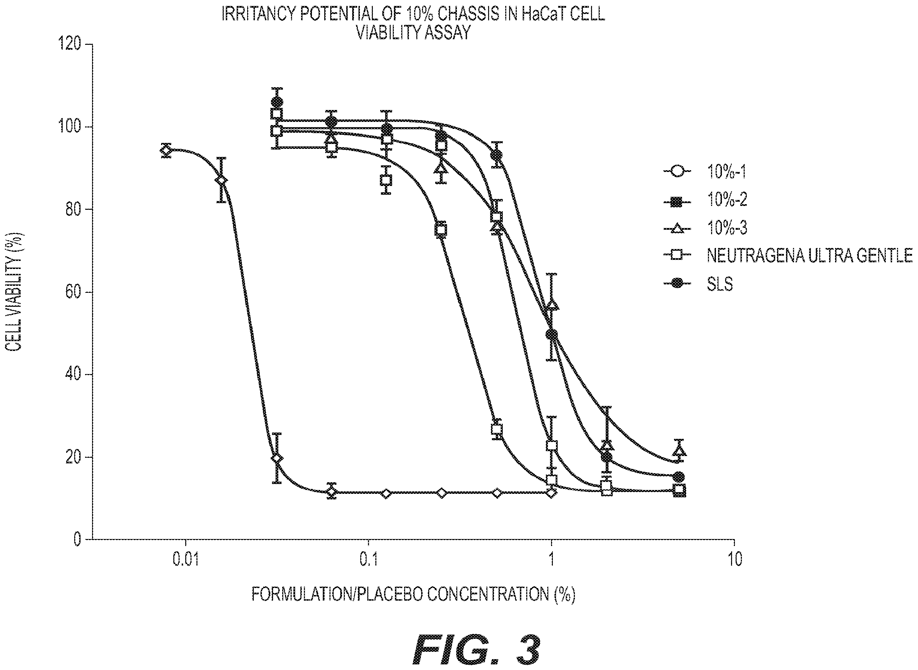

Cell viability studies were conducted to assess the skin irritation of the formulations using Neutrogena Ultra Gentle Daily Cleanser and PanOxyl 10 (Placebo) as the comparators. The results are shown in Figure 2 for the 2.5% BPO formulations and Figure 3 for the 10% BPO formulations. Sodium Lauryl Sulfonate (SLS) was used as the positive control. All of the 6 tested formulations were found to be extremely mild. All 6 formulations outperformed Neutrogena Ultra Gentle with formulation #3(2.5%) being more than 3 fold milder than Neutrogena Ultra Gentle cleanser. The 2.5% prototypes were slightly milder than the 10% formulations. This is expected due to higher concentration of the surfactants in the 10% formulations.

For purposes herein Neutrogena Ultra Gentle has an ingredient list of Water, Glycerin, Cocamidopropyl Betaine, Lauryl Glucoside, Potassium Acrylates Copolymer, PEG-120 Methyl Glucose Dioleate, Disodium Lauroamphodiacetate, Sodium Cocoyl Sarcosinate, Ethylhexylglycerin, Caprylyl Glycol, Potassium Sorbate, and Fragrance.

### Physical stability

All the formulations passed a stress centrifuge test at 2250 g for 30 minutes. The formulations were stored at 25°C/60% RH and 30°C/65% RH for 3 months, and no discoloration or phase separation were observed. Commonly, the pH values of the BPO based cleansers decrease over time due to BPO degradation and subsequent benzoic acid generation. Buffer systems have been utilized but they could have a negative effect on BPO stability, viscosity, and foaming.

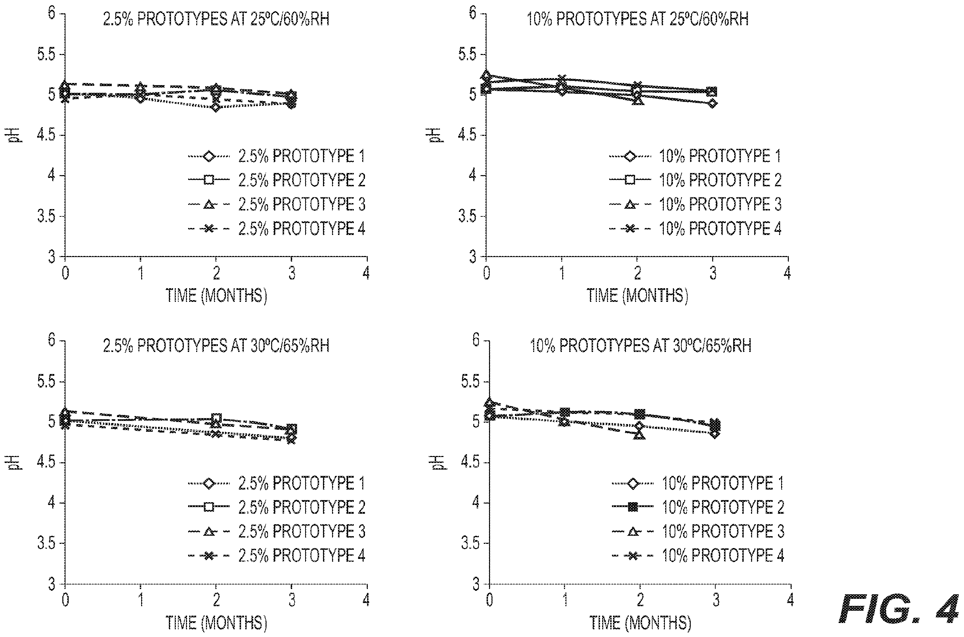

Figure 4 shows the pH values of the 2.5% and 10% formulations on storage. There was a minimum pH shift at 25°C/60% RH after 3 months. The pH decreased less than 0.13 units for 2.5% formulations and less than 0.19 units for 10% formulations #1, # 2 and #4. The pH of 10% Formulation # 3 decreased by 0.3 units after 2 months. As expected samples at 30°C/65% RH showed slightly larger change in pH due to higher rate of BPO degradation. The 2.5% formulations showed less than 0.2 unit decrease in pH after 3 months.

Formulations # 1, #2 and #4 of the 10% BPO showed less than 0.22 unit decrease in pH after 3 months. The 10% formulation # 3 showed the highest change in pH with 0.4 unit decrease after 2 months. International Conference on Harmonization (ICH guidelines) were followed in this method.

### Chemical stability

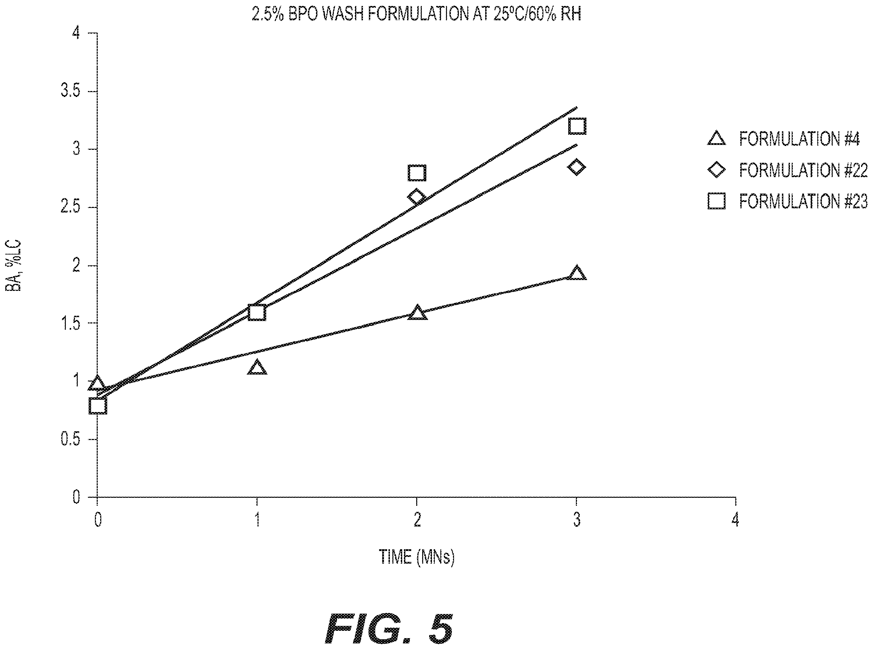

Chemical stability of the BPO in surfactant based wash products is a challenge due to increased solubility of the BPO in these systems. Based on binary and solubility studies, a Suganate 160 NC and Plantaren 2000 N UP surfactant system with or without Sorbitol was chosen for further study. The four 2.5% BPO formulations (formulations #1-4) were tested and are expected to have a shelf life prediction of greater than 24 months. The four 10% formulations were tested at 25°C/60% RH and are expected to have a shelf life prediction of greater than 81months. At 30°C/65% RH, the predicted shelf-life is greater than 27 months. Figure 5 demonstrates the stability data of 2.5% BPO wash formulation under 25 C° /60 RH conditions. This data shows the superiority of the formulations with sugar based surfactants (formulation #4) as compared to formulations that do not contain sugar based surfactants, formulations # 22 and #23. The predicted shelf lives of these formulations (# 22 and #23 & # 4) respectively are 13 months, 11 months and 27 months.

### In-vitro Efficacy against P. Acnes

*In vitro* efficacy of selected formulations (Table 6) against *P.acne* was evaluated in vitro. P. *Acne* contaminated human donated skin were treated with the wash formulations for 3mins and then rinsed off. Subsequently, the bacteria were scraped off and incubated in media for 3 and 5 days at 37°C under anaerobic conditions. More than 5 log reduction (>99.999%) on P. *Acnes* was observed with the 3 day data and 3-5 log reduction (99.9%- 99.999%) was also observed with the 5 day data.

**Table 6 In vitro efficacy of formulations against P. Acne**

| Treatment | Mean count (CFU/ml) | |
|---|---|---|
| | 3 day | 5day |
| Control | 1.6 x 10⁶ | 1.6 x 10⁶ |
| PanOxyl10 (10% BPO) | ≤1.0 x 10¹ | ≤1.0 x 10¹ |
| #11 - 10% BPO | ≤1.0 x 10¹ | 1.1 x 10³ |
| #11 (repeat) | ≤1.0 x 10¹ | 1.9 x 10² |
| #22 2.5% BPO | ≤1.0 x 10¹ | 1.0 x 10¹ |
| #24- 2.5% BPO | ≤1.0 x 10¹ | ≤1.0 x 10¹ |

## Claims

1. A topical pharmaceutical or cosmetic cleanser composition comprising
a) benzoyl peroxide present in an amount from about 2.0% to about 20% w/w;
b) water,
c) at least one anionic surfactant which is a alkyl glucoside (AG) derivative surfactant selected from an alkylglycoside sulfonate, an alkylpolyglycoside sulfonate, or an alkylpolyglycoside phosphate;
d) at least one humectant,
e) a thickening agent, and
f) optionally at least one dermatologically acceptable excipient.

2. The composition according to claim 1 further comprising a coemulsifier.

3. The composition according to claim 1 or 2 wherein the thickening agent is selected from the group consisting of agar, an alginate, sodium alginate, arabinoxylan, carrageenan, celluloses and derivatives thereof, carboxymethylcellulose, hydroxyethylcellulose, hydroxypropylcellulose, hydroxypropyl methylcellulose, cellulose, curdlan, gelatin, gellan, β-glucan, tragacanth gum, gum arabic, pectin, starch, modified starches, modified potato starch, modified corn starch, a carbomer and salts thereof, an acrylate copolymer, silica, a polysaccharide, xanthan gum and dehydroxanthan gum and salts thereof, guar gum, locust bean gum, gum tragacanth and extracts of quince seeds, or a combination or mixture thereof.

4. The composition according to any one claims 1 to 3 wherein the thickening agent is selected from a carbomer or a salt thereof, an acrylate copolymer, polyacrylate crosspolymer-6.

5. The composition according to any of the preceding claims wherein the at least one humectant is selected from glycerol, sorbitol or a combination of glycerol and sorbitol.

6. The composition according to any of the preceding claims wherein the AG derivative surfactant is selected from Sodium Laurylglucoside Hydroxypropylsufonate, Sodium Decylglucoside Hydroxypropylsufonate, Sodium Hydroxypropylphosphate Laurylglucoside Crosspolymer, or Sodium Hydroxypropylphosphate Decylglucoside Crosspolymer, or a combination or mixture thereof.

7. The composition according to any of claims 1 to 6 further comprising an additional surfactant selected from a second anionic surfactant, or a non-ionic surfactant.

8. The composition according to claim 7 wherein the second anionic surfactant is selected from sodium C14-16 Olefin sulfonate, disodium PEG-12 Dimethicone Sulfosuccinate, ammonium lauryl sulfate, sodium lauroyl methyl isoethionate , Sodium C14-C17 sec-alkyl sulfonate, or disodium laureth sulfosuccinate, and mixtures thereof.

9. The composition according to claim 7 wherein the nonionic surfactant is selected from decyl glucoside or cocoglycoside or a mixture thereof.

10. The composition according to any of the preceding claims wherein the composition comprises at least one AG derivative surfactant, as defined in claim 1, at least one second anionic surfactant and at least one non-ionic surfactant.

11. The composition according to any of the preceding claims wherein the benzoyl peroxide present in an amount from 4.0 to 10% w/w.

12. The composition according to any of the preceding claims which further comprises an additional dermatologically acceptable excipient selected from a pH adjusting agent, a chelating agent, a preservative, a co-solvent, a penetration enhancer, a fragrance, a colorant, and mixtures thereof.

13. A topical pharmaceutical or cosmetic cleanser composition according to any of the preceding claims which has a shelf life of greater than 18 months under ICH conditions.

14. A topical pharmaceutical or cosmetic cleanser composition according to any of the preceding claims which has a shelf life of greater than 24 months under ICH conditions.

## Patentansprüche

1. Topische pharmazeutische oder kosmetische Reinigungszusammensetzung, umfassend
a) Benzoylperoxid, das in einer Menge von etwa 2,0% bis etwa 20% w/w enthalten ist;
b) Wasser,
c) mindestens ein anionisches Tensid, das ein Tensid eines Alkylglucosid (AG)-Derivats ist, ausgewählt aus einem Alkylglycosidsulfonat, einem Alkylpolyglycosidsulfonat oder einem Alkylpolyglycosidphosphat;
d) mindestens ein Feuchthaltemittel,
e) ein Verdickungsmittel, und
f) optional mindestens einen dermatologisch verträglichen Exzipienten.

2. Zusammensetzung gemäß Anspruch 1, ferner umfassend einen Coemulgator.

3. Zusammensetzung gemäß Anspruch 1 oder 2, worin das Verdickungsmittel ausgewählt ist aus der Gruppe bestehend aus Agar, einem Alginat, Natriumalginat, Arabinoxylan, Carrageenan, Cellulosen und Derivaten davon, Carboxymethylcellulose, Hydroxyethylcellulose, Hydroxypropylcellulose, Hydroxypropylmethylcellulose, Cellulose, Curdlan, Gelatine, Gellan, β-Glucan, Tragantgummi, Gummi arabicum, Pektin, Stärke, modifizierten Stärken, modifizierter Kartoffelstärke, modifizierter Maisstärke, einem Carbomer und Salzen davon, einem Acrylatcopolymer, Siliciumdioxid, einem Polysaccharid, Xanthangummi und Dehydroxanthangummi und Salzen davon, Guargummi, Johannisbrotkernmehl, Tragantgummi und Extrakten aus Quittensamen oder einer Kombination oder Mischung davon.

4. Zusammensetzung gemäß einem der Ansprüche 1 bis 3, worin das Verdickungsmittel ausgewählt ist aus einem Carbomer oder einem Salz davon, einem Acrylatcopolymer, einem Polyarcylat-Kreuzpolymer-6.

5. Zusammensetzung gemäß einem der vorangehenden Ansprüche, worin das mindestens eine Feuchthaltemittel ausgewählt ist aus Glycerin, Sorbitol oder einer Kombination aus Glycerin und Sorbitol.

6. Zusammensetzung gemäß einem der vorangehenden Ansprüche, worin das Tensid eines Alkylglucosid (AG)-Derivats ausgewählt ist aus Natrium-Laurylglucosid-Hydroxypropylsulfonat, Natrium-Decylglucosid-Hydroxypropylsulfonat, Natrium-Hydroxypropylphosphat-Laurylglucosid-Kreuzpolymer oder Natrium-Hydroxypropylphosphat-Decylglucosid-Kreuzpolymer oder einer Kombination oder Mischung davon.

7. Zusammensetzung gemäß einem der Ansprüche 1 bis 6, ferner umfassend ein zusätzliches Tensid ausgewählt aus einem zweiten anionischen Tensid oder einem nichtionischen Tensid.

8. Zusammensetzung gemäß Anspruch 7, worin das zweite anionische Tensid ausgewählt ist aus Natrium-C14-16-Olefinsulfonat, Dinatrium-PEG-12-Dimethicon-Sulfosuccinat, Ammoniumlaurylsulfat, Natriumlauroylmethylisoethionat, Natrium-C14-C17-sek-Alkylsulfonat oder Dinatriumlaurethsulfosuccinat und Mischungen davon.

9. Zusammensetzung gemäß Anspruch 7, worin das nichtionische Tensid ausgewählt ist aus Decylglucosid oder Cocoglycosid oder einer Mischung davon.

10. Zusammensetzung gemäß einem der vorangehenden Ansprüche, worin die Zusammensetzung mindestens ein Tensid eines AG-Derivats, wie in Anspruch 1 definiert, mindestens ein zweites anionisches Tensid und mindestens ein nichtionisches Tensid umfasst.

11. Zusammensetzung gemäß einem der vorangehenden Ansprüche, worin das Benzolyperoxid in einer Menge von 4,0 bis 10% w/w enthalten ist.

12. Zusammensetzung gemäß einem der vorangehenden Ansprüche, die ferner einen zusätzlichen dermatologisch verträglichen Exzipienten umfasst, der aus einem pHeinstellenden Mittel, einem Chelatbildner, einem Konservierungsmittel, einem Co-Lösungsmittel, einem Penetrationsverstärker, einem Duftstoff, einem Farbstoff und Mischungen davon ausgewählt ist.

13. Topische pharmazeutische oder kosmetische Reinigungszusammensetzung gemäß einem der vorangehenden Ansprüche, die unter ICH-Bedingungen eine Haltbarkeit von mehr als 18 Monaten aufweist.

14. Topische pharmazeutische oder kosmetische Reinigungszusammensetzung gemäß einem der vorangehenden Ansprüche, die unter ICH-Bedingungen eine Haltbarkeit von mehr als 24 Monaten aufweist.

## Revendications

1. Composition nettoyante pharmaceutique ou cosmétique topique comprenant
a) du peroxyde de benzoyle présent en une quantité d'environ 2,0 % à environ 20 % p/p;
b) de l'eau,
c) au moins un tensioactif anionique qui est un tensioactif dérivé d'alkylglucoside (AG) choisi parmi un alkylglycoside sulfonate, un alkylpolyglycoside sulfonate, ou un alkylpolyglycoside phosphate;
d) au moins un humectant,
e) un agent épaississant, et
f) éventuellement au moins un excipient dermatologiquement acceptable.

2. Composition selon la revendication 1 comprenant en outre un co-émulsifiant.

3. Composition selon la revendication 1 ou 2 dans laquelle l'agent épaississant est choisi dans le groupe consistant en la gélose, un alginate, l'alginate de sodium, l'arabinoxylane, le carraghénane, les celluloses et leurs dérivés, la carboxyméthylcellulose, l'hydroxyéthylcellulose, l'hydroxypropylcellulose, l'hydroxypropylméthylcellulose, la cellulose, le curdlane, la gélatine, la gellane, le β-glucane, la gomme adragante, la gomme arabique, la pectine, l'amidon, les amidons modifiés, l'amidon de pomme de terre modifié, l'amidon de maïs modifié, un carbomère et ses sels, un copolymère d'acrylate, la silice, un polysaccharide, la gomme de xanthane et la gomme de déshydroxanthane et leurs sels, la gomme de guar, la gomme de caroube, la gomme adragante et les extraits de graines de coing, ou une combinaison ou un mélange de ceux-ci.

4. Composition selon l'une quelconque des revendications 1 à 3 dans laquelle l'agent épaississant est choisi parmi un carbomère ou un sel de celui-ci, un copolymère d'acrylate, un polymère réticulé de polyacrylate 6.

5. Composition selon l'une quelconque des revendications précédentes dans laquelle le au moins un l'humectant est choisi parmi le glycérol, le sorbitol ou une combinaison de glycérol et de sorbitol.

6. Composition selon l'une quelconque des revendications précédentes dans laquelle le tensioactif dérivé d'AG est choisi parmi le laurylglucoside hydroxypropylsufonate de sodium, le décylglucoside hydroxypropylsufonate de sodium, un polymère réticulé d'hydroxypropylphosphate de sodium et de laurylglucoside, ou un polymère réticulé d'hydroxypropylphosphate de sodium et de décylglucoside, ou une combinaison ou un mélange de ceux-ci.

7. Composition selon l'une quelconque des revendications 1 à 6 comprenant en outre un tensioactif supplémentaire choisi parmi un second tensioactif anionique, ou un tensioactif non ionique.

8. Composition selon la revendication 7 dans laquelle le second tensioactif anionique est choisi parmi le C14-16 oléfine sulfonate de sodium, le PEG-12 diméthicone sulfosuccinate de disodium, le laurylsulfate d'ammonium, le lauroylméthyliséthionate de sodium, le C14-C17 sec-alkylsulfonate de sodium, ou le laureth sulfosuccinate de disodium, et leurs mélanges.

9. Composition selon la revendication 7 dans laquelle le tensioactif non ionique est choisi parmi le décylglucoside ou le cocoglycoside ou un mélange de ceux-ci.

10. Composition selon l'une quelconque des revendications précédentes dans laquelle la composition comprend au moins un tensioactif dérivé d'AG, tel que défini dans la revendication 1, au moins un second tensioactif anionique et au moins un tensioactif non ionique.

11. Composition selon l'une quelconque des revendications précédentes dans laquelle le peroxyde de benzoyle est présent en une quantité de 4,0 à 10 % p/p.

12. Composition selon l'une quelconque des revendications précédentes, qui comprend en outre un excipient dermatologiquement acceptable supplémentaire choisi parmi un agent d'ajustement du pH, un agent chélatant, un conservateur, un cosolvant, un activateur de pénétration, un parfum, un colorant et leurs mélanges.

13. Composition nettoyante pharmaceutique ou cosmétique topique selon l'une quelconque des revendications précédentes qui a une durée de conservation supérieure à 18 mois dans des conditions ICH.

14. Composition nettoyante pharmaceutique ou cosmétique topique selon l'une quelconque des revendications précédentes qui a une durée de conservation supérieure à 24 mois dans des conditions ICH.
